# FASCICULE DE BREVET EUROPEEN

(11) **EP 2 986 983 B1**
(45) Date de publication et mention de la délivrance du brevet: **22.05.2019**
(21) Numéro de dépôt: 14718599.5
(22) Date de dépôt: 22.04.2014
(51) Int. Cl.: G01N 33/50, G01N 33/68

(54) **UTILISATION DE BIOMARQUEURS DE LA BARRIÈRE POUR L'ÉVALUATION DE L'EFFICACITÉ D'ACTIFS**
VERWENDUNG VON BARRIEREBIOMARKERN ZUR BEWERTUNG DER WIRKSAMKEIT VON WIRKSTOFFEN
USE OF BARRIER BIOMARKERS FOR EVALUATING THE EFFECTIVENESS OF ACTIVE INGREDIENTS

(30) Priorité: 19.04.2013 FR 1353632
(43) Date de publication de la demande: 24.02.2016
(73) Titulaire: Laboratoires Expanscience, 92048 Paris La Défense Cedex (FR)
(72) Inventeur: MSIKA, Philippe, F-78000 Versailles (FR); BAUDOUIN, Caroline, F-78120 Rambouillet (FR); BREDIF, Stéphanie, F-28120 Chaudon (FR)
(74) Mandataire: Regimbeau
(86) Numéro de dépôt international: PCT/EP2014/058042
(87) Numéro de publication internationale: WO 2014/170495

(56) Documents cités:
- FR-A1- 2 968 201
- US-A1- 2005 191 709
- US-A1- 2010 035 816
- US-A1- 2011 262 025
- US-A1- 2012 184 448
- "Avocado sugars are effective inducer of cutaneous defensive functions", JOURNAL OF THE AMERICAN ACADEMY OF DERMATOLOGY, C.V. MOSBY, ST. LOUIS, MO, US, vol. 56, no. 2, 1 février 2007 (2007-02-01), page AB84, XP005937005, ISSN: 0190-9622
- BREDIF S ET AL: "AVOCADO SUGARS ARE EFFECTIVE INDUCERS OF CUTANEOUS DEFENSIVE FUNCTIONS", THE JOURNAL OF INVESTIGATIVE DERMATOLOGY, NATURE PUBLISHING GROUP, GB, vol. 126, no. SUPPL 3, 7 septembre 2006 (2006-09-07), page 52, XP009081682, ISSN: 0022-202X
- "Antiitching properties of patented avocado peptides", JOURNAL OF THE AMERICAN ACADEMY OF DERMATOLOGY, C.V. MOSBY, ST. LOUIS, MO, US, vol. 62, no. 3, 1 mars 2010 (2010-03-01), page AB54, XP026902633, ISSN: 0190-9622, DOI: 10.1016/J.JAAD.2009.11.597 [extrait le 2010-02-13]
- RAWLINGS A V: "Trends in stratum corneum research and the management of dry skin conditions", INTERNATIONAL JOURNAL OF COSMETIC SCIENCE, KLUWER ACADEMIC PUBLISHERS, DORDRECHT, NL, vol. 25, no. 1-2, 1 janvier 2003 (2003-01-01), pages 63-95, XP009097795, ISSN: 0142-5463, DOI: 10.1046/J.1467-2494.2003.00174.X
- S Leclere-Bienfait ET AL: "Avocado perseose, a biomimetic active ingredient for the protection and accompaniment of infants' skin", Journal of investigative dermatology, 1 mai 2013 (2013-05-01), page S106, XP055095186, DOI: http://www.nature.com/jid/journal/v133/n1s /index.html#ab Extrait de l'Internet: URL:http://www.nature.com/jid/journal/v133 /n1s/pdf/jid201399a.pdf [extrait le 2014-01-08]

## Description

La présente invention se rapporte au domaine du théranostique, aussi parfois appelé théragnostique, c'est-à-dire à l'évaluation de l'efficacité d'actifs ou de composés dans le traitement de sujets, en particulier en vue d'adapter leur traitement de façon individuelle, pour une médecine personnalisée. Plus précisément, l'invention appartient au domaine de la dermatologie, et porte sur des méthodes pour évaluer l'efficacité d'un actif choisi parmi les sucres en C7 et les dérivés de formule (I), dans la prévention et/ou le traitement d'au moins une déficience de la barrière cutanée d'un sujet.

La peau protège l'organisme contre des facteurs externes de contraintes physiques (facteurs mécaniques, thermiques et rayons UV, notamment), chimiques (tensioactifs, exposition prolongée à l'eau, solvants, etc.) et environnementaux. De plus, la peau, en tant que barrière, protège l'organisme contre la perte d'ions essentiels, d'eau et de protéines sériques.

L'épiderme constitue la couche la plus superficielle de la peau et assure l'imperméabilité et la résistance de celle-ci. On peut identifier dans l'épiderme 4 couches cellulaires distinctes, une couche basale (*stratum basalis*), une couche épineuse (*stratum spinosum*), une couche granuleuse (*stratum granulosum*), et une couche cornée (*stratum corneum*).

Parmi elles, ce sont en particulier la couche cornée (*stratum corneum*) et la couche granuleuse (*stratum granulosum*), c'est-à-dire les couches les plus externes de l'épiderme, qui assurent la fonction barrière de la peau.

La couche cornée est majoritairement formée de kératinocytes cornifiés, aussi appelés cornéocytes. Les kératinocytes cornifiés sont des cellules ayant perdu leur noyau cellulaire qui résultent d'un processus continu de différentiation des kératinocytes, aussi appelé maturation kératinocytaire ou maturation épidermique. Les kératinocytes cornifiés sont reliés entre eux par des desmosomes, et s'organisent en un réseau de cellules accolées les unes aux autres et formant des lamelles souples et résistantes. Entre les kératinocytes cornifiés, le domaine intercellulaire est riche en lipides, en particulier ceux de la famille des céramides. En outre, localisés dans la couche cornée, se trouvent des "facteurs d'hydratation naturels" (naturel moisturizing factors NMF), qui maintiennent le degré d'hydratation de la peau à un niveau optimal.

Cette barrière réalisée par la couche cornée n'est pas absolue. En effet, il existe une perte transépidermique d'eau, ou perte insensible en eau, qui est minime, mais qui peut augmenter dans certaines situations de déficience de la fonction barrière. La fonction barrière de la peau évolue avec l'âge du sujet, et peut être incomplète, voire déficiente, à certains âges de la vie.

Il est connu par exemple que la peau de l'enfant n'est pas mature, et que sa fonction barrière évolue avec sa maturation. En particulier, la barrière de la peau des enfants n'est qu'incomplètement fonctionnelle en comparaison de la peau d'adulte.

Fluhr et al (Br J Dermatol 166 (3):483-90, 2012) ont ainsi montré que la teneur en eau de la couche cornée est plus faible à la naissance (enfants âgés de 1 à 15 jours) que chez l'adulte. Les prématurés en particulier présentent un accroissement de perte insensible d'eau (les pertes insensibles en eau sont multipliées par 10 environ chez les prématurés) et une sensibilité élevée aux infections, témoignant de l'immaturité de la barrière cutanée dans cette population de sujets.

D'autre part, la peau du sujet âgé présente elle aussi une diminution de la barrière cutanée, liée au phénomène de sénescence, aussi appelé vieillissement intrinsèque (J. Lübbe, Revue Médicale Suisse, 62(2472) : 488-490, 2004).

La barrière cutanée peut aussi être affaiblie du fait d'altérations de sa structure, celles-ci pouvant résulter tant de facteurs endogènes que de facteurs exogènes. On estime ainsi par exemple que l'acné du nourrisson, l'acné de l'adolescent, la rosacée ou érythrocouperose, le psoriasis, la dermite du siège, la dermatite atopique, l'eczéma, la dermatite de contact, la dermatite irritative et en particulier la dermatite irritative du siège ou érythème fessier, la dermatite allergique, la dermite séborrhéique, la sécheresse cutanée, l'hyperréactivité cutanée pour ne citer que ces pathologies, sont liées à une déficience de la barrière cutanée.

Des actifs permettant de prévenir ou de restaurer la fonction barrière de la peau sont déjà connus dans l'art. En particulier, l'effet protecteur pour la fonction barrière de sucres en C7 et leurs dérivés, le perséose d'avocat, a déjà été décrit (voir par exemple WO 2005/105123).

L'effet d'un mélange de sucres d'avocat sur l'expression de marqueurs de la maturation épidermique par des cellules de peau adulte a été décrit (Bredif et al., 2006, J Invest Dermatol, 26(3), 52 ; Bredif et al., 2007, J Am Acad Dermatol, 56(2), AB84). Des peptides d'avocat ont également été décrits (Bredif et al., 2010, J Am Acad Dermatol, 62(3), AB54 ; US2010/0035816).

Toutefois, il n'était jusqu'alors pas connu de marqueur biologique permettant de mesurer l'efficacité dudit perséose d'avocat durant un traitement, chez un sujet particulier. Or, l'efficacité d'un traitement dermatologique peut dépendre de l'état de la peau du sujet traité. De ce fait, il n'était pas possible jusqu'à présent d'adapter ou d'ajuster ledit traitement en fonction du sujet traité.

Les présents inventeurs ont, pour la première fois, identifié des marqueurs qui sont spécifiquement exprimés ou activés quand la peau est traitée avec le perséose d'avocat. Ils ont en particulier observé que certains marqueurs biologiques sont plus exprimés ou plus actifs dans des peaux traitées au perséose d'avocat que dans des peaux non traitées. Ces marqueurs peuvent ainsi être utilisés pour évaluer l'efficacité du perséose d'avocat pour prévenir ou traiter une déficience de la barrière chez un sujet.

### Description détaillée

Il est ici décrit une méthode pour évaluer l'efficacité d'un actif choisi parmi les sucres d'avocat en C7, aussi appelés perséose d'avocat et que l'on définira plus loin, pour prévenir ou traiter une déficience de la barrière cutanée, ladite méthode comprenant la détermination du niveau d'expression et/ou d'activation d'au moins un marqueur biologique.

Il est bien entendu que les actifs sont utilisés chez des mammifères, et de préférence des humains. De façon plus préférentielle, lesdites compositions sont utilisées chez des enfants. Par « sujet », on entend ici tout sujet humain, quel que soit son âge. Ainsi, le sujet peut être par exemple un adulte ou un enfant. Par « enfant », on entend ici un individu dont l'âge est inférieur à 16 ans. Sont ainsi compris dans la catégorie des enfants, les nouveau-nés, c'est-à-dire les enfants dont l'âge est compris entre 0 et 1 mois, les nourrissons, c'est-à-dire les enfants dont l'âge est compris entre 1 mois et 2 ans, et les enfants de 2 ans ou plus, c'est-à-dire les enfants dont l'âge est compris entre 2 ans et 16 ans. Un « nouveau-né », comme on l'entend ici, peut aussi bien être né à terme qu'être prématuré.

Pour lever toute ambiguïté, le terme enfant utilisé dans la présente demande sans autre précision doit être entendu dans son acception la plus générale, c'est-à-dire comme se référant à une personne de moins de 16 ans. Ainsi, dans le cadre de la présente méthode, les enfants âgés de 2 à 16 ans sont désignés spécifiquement par les termes « enfants de 2 ans ou plus ».

Par « adulte » on entend ici une personne qui n'est pas un enfant, autrement dit une personne âgée de plus de 16 ans. En particulier, le sujet est un enfant, préférentiellement un nouveau-né ou un nourrisson. Dans l'invention, le sujet est un enfant, préférentiellement un nouveau-né ou un nourrisson.

Il est ici décrit une méthode pour évaluer l'efficacité d'un actif choisi parmi les sucres en C7 et les dérivés de formule (I), 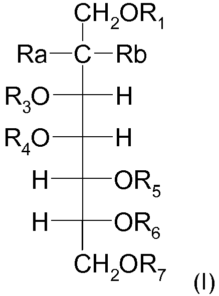 où
Ra représente un atome d'hydrogène et Rb représente un -OR2 ou CRaRb représente le radical CO ;
R1, R2, R3, R4, R5, R6 et R7 représentent, indépendamment l'un de l'autre :
   - un atome d'hydrogène ou
   - un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique ; ou
   - un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique ;
dans la prévention et/ou le traitement d'au moins une déficience de la barrière cutanée d'un sujet, ladite méthode comprenant les étapes de:
a. mesure du niveau d'expression et/ou d'activation d'au moins un marqueur biologique dans un échantillon de cellules cutanées du sujet, caractérisé en ce que ledit marqueur biologique est choisi parmi:
   - les marqueurs de la maturation épidermique, ledit marqueur de la maturation épidermique étant de préférence choisi parmi la desmogléine 1 et l'involucrine, ou ;
   - les marqueurs de la barrière lipidique, ledit marqueur de la barrière lipidique étant de préférence choisi parmi les céramides, en particulier parmi les céramides 1 à 9, encore plus particulièrement le céramide 1, ou ;
   - les marqueurs de la régulation hydrique, ledit marqueur de la régulation hydrique étant de préférence choisi parmi la filaggrine, PAD1, l'acide hyaluronique et la transglutaminase 1, ou ;
   - les marqueurs de la régulation du *stratum granulosum,* ledit marqueur de la régulation du *stratum granulosum* étant de préférence choisi parmi les claudines, en particulier la claudine 1 ;
b. mesure du niveau d'expression et/ou d'activation dudit marqueur biologique dans un échantillon de cellules cutanées de référence ;
c. comparaison des niveaux d'expression et/ou d'activation obtenus à l'étape a) avec des niveaux d'expression et/ou d'activation obtenus à l'étape b);
d. d'évaluation de l'efficacité dudit actif en fonction de la comparaison de l'étape b).

L'invention concerne en particulier une méthode telle que revendiquée dans les revendications.

Comme il est bien connu de l'homme du métier, la fonction barrière de la peau correspond à la capacité de la peau de limiter les échanges entre le milieu extérieur et l'intérieur du corps, plus particulièrement la diffusion d'eau. L'« intégrité de la fonction barrière de la peau » ou « intégrité de la barrière cutanée » (telles qu'entendues ici, les deux expressions sont synonymes) signifie donc que la barrière cutanée est pleinement fonctionnelle, c'est-à-dire que les échanges, et en particulier la diffusion d'eau, sont limités.

L'intégrité de la barrière cutanée peut être évaluée par la mesure d'un grand nombre de paramètres. En particulier, il est habituel de déterminer l'intégrité de la barrière cutanée par la mesure de la perte insensible en eau, aussi appelée perte en eau transépidermique. Les méthodes de mesure de la perte insensible en eau sont bien connues de l'homme de l'art et ne nécessitent pas d'être décrites ici en détail (voir par exemple H Tagami and K Kikuchi. « Diseases that affect barrier function ». In Elias PM and Feingold KR editors. Skin Barrier. New York: Taylor and Francis; 2005. p447-468). Préférentiellement, pour mesurer la perte insensible en eau, on utilise un évaporimètre, qui est composé d'une sonde que l'on place à 3 ou 6 mm au-dessus de la peau.

Le D-mannoheptulose, premier cétoheptose identifié en 1916 par La Forge, de formule générale (II) 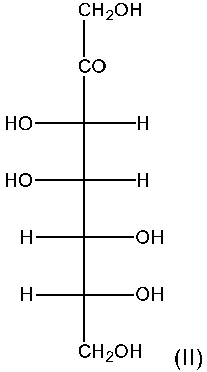 est un sucre en C7 qui se retrouve dans certaines plantes, en particulier dans la luzerne (Medicago sativa L.), dans l'avocat, dans la figue (Ficus officinalis L.) dans l'orpin (Sedum spectabile Bor.) et dans la primevère (Primula officinalis Jacq.). Toutefois, c'est dans l'avocat, que l'on retrouve les teneurs les plus importantes en D-mannoheptulose. Le D-mannoheptulose a déjà été utilisé dans des applications thérapeutiques. Par exemple, la demande de brevet WO 95/03809 décrit l'utilisation du D-mannoheptulose, en tant qu'inhibiteur de glucokinase, pour inhiber le développement des cellules tumorales et la demande US 2003/0092669 décrit un complément alimentaire oral comprenant du D-mannoheptulose, qui permet de diminuer le taux d'insuline et qui permet ainsi une perte de poids.

Le perséitol, forme polyol du D-mannoheptulose, de formule générale (III) 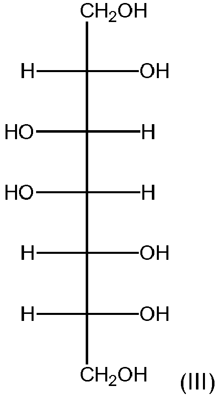 se retrouve également dans l'avocat, en particulier dans le fruit ou dans le noyau de l'avocat. Il a été montré que le perséitol, associé à un ion potassium, permet d'inhiber l'incorporation de leucine-3H dans des cellules tumorales d'ascite sarcomateuse d'Ehrlich (Shibuya et al., Pure Appl. Chem., 71(6) : 1109-1113, 1999). L'utilisation de ces sucres (perséitol et D-mannoheptulose) pour traiter l'alopécie a déjà été décrite (WO 2011/073281). L'utilisation du perséose d'avocat dans le traitement de candidoses et de pityrosporoses a également déjà été décrite (WO 2008/025847). Il avait enfin été montré que ces sucres pouvaient être utilisés pour stimuler la synthèse des beta-défensines humaines (en particulier HBD-2) (WO 2005/115421, WO 2005/105123). En particulier, ces sucres étaient déjà connus pour être efficaces dans la prévention ou le traitement des pathologies liées à un déficit de la barrière cutanée (voir par exemple WO 2005/105123).

En revanche, il n'avait jusqu'alors pas été mis en évidence de marqueurs biologiques dont l'expression ou l'activation était corrélée avec l'utilisation de ces sucres dans le traitement des déficiences de la barrière cutanée. Or les présents inventeurs ont identifié de tels marqueurs biologiques, ce qui permet d'évaluer l'efficacité desdits sucres dans la prévention ou le traitement des déficiences de la barrière cutanée d'un sujet.

Par « efficacité d'un actif dans la prévention d'au moins une déficience de la barrière cutanée d'un sujet », on entend ici la capacité dudit actif à maintenir l'intégrité fonctionnelle de la barrière cutanée chez le sujet. Autrement dit, l'efficacité d'un actif dans la prévention d'au moins une déficience de la barrière cutanée d'un sujet correspond à la capacité dudit actif d'empêcher la détérioration de la fonction barrière de la peau dudit sujet.

Par « efficacité d'un actif dans le traitement d'au moins une déficience de la barrière cutanée d'un sujet », on entend ici la capacité dudit actif à restaurer l'intégrité fonctionnelle de la barrière cutanée chez le sujet. Autrement dit, l'efficacité d'un actif dans le traitement d'au moins une déficience de la barrière cutanée d'un sujet correspond à la capacité dudit actif de renverser la détérioration de la fonction barrière de la peau dudit sujet. Par « marqueur biologique », on entend ici une caractéristique qui est objectivement mesurée et évaluée comme indicateur de processus biologiques normaux, de processus pathogéniques, ou de réponses pharmacologiques à une intervention thérapeutique. Un marqueur biologique désigne donc toute une gamme de substances et de paramètre divers. Par exemple, un marqueur biologique peut être une substance dont la détection indique un état pathologique particulier (par exemple la présence de la protéine C activée en tant que marqueur d'une infection), ou au contraire une substance dont la détection indique un état physiologique spécifique. Le marqueur biologique de la présente méthode est préférentiellement un gène, les produits d'un gène tels que ses transcrits et les peptides issus de ses transcrits, un lipide, un sucre ou un métabolite.

Par exemple, le marqueur biologique est un gène, les produits d'un gène tels que des transcrits ou des peptides, un lipide, un sucre ou un métabolite dont les changements d'expression, en particulier de niveau d'expression, corrèlent avec un état physiologique de la barrière cutanée.

Par exemple, le marqueur biologique est un gène, les produits d'un gène tels que des transcrits ou des peptides, un lipide, un sucre ou un métabolite dont les changements d'activation, en particulier de niveau d'activation, corrèlent avec un état physiologique de la barrière cutanée. En particulier, le marqueur biologique est un peptide ayant une activité enzymatique.

Le marqueur biologique de la présente méthode est choisi parmi les marqueurs de la maturation épidermique, les marqueurs de la barrière lipidique, les marqueurs de la régulation hydrique, et les marqueurs de la régulation du *stratum granulosum.* Les inventeurs ont en particulier mis en évidence qu'un traitement au perséose d'avocat conduit à une augmentation de l'expression de marqueurs clés de la maturation épidermique tels que la desmogléine et l'involucrine dans des modèles de de peau *in vitro.*

La protéine involucrine est exprimée dans les couches épineuses-granuleuses. C'est le premier précurseur de l'enveloppe cornée qui représente 5 à 15% de l'enveloppe cornée, et sert de lien également avec l'enveloppe cornée lipidique. La protéine desmogléine est synthétisée par les kératinocytes et sécrétée à l'interface *stratum granulosum-stratum corneum.* Elle est un composant indispensable des cornéodesmosomes, les jonctions qui lient les cornéocytes.

La mesure de l'expression de ces marqueurs chez le sujet traité par l'actif de la méthode permet donc de mesurer que celui-ci est bien efficace chez ledit sujet, en particulier sur la maturation épidermique.

Dans l'invention, le marqueur de la maturation épidermique est choisi parmi la desmogléine 1 et l'involucrine.

Au sens de la présente méthode, le marqueur biologique desmogléine 1 comprend le gène DSG1 humain (référence NCBI : GENE ID 1828), ainsi que les produits de ce gène. Les produits du gène DSG1 humain comprennent le transcrit du gène DSG1 humain et le peptide précurseur de la protéine desmogléine 1 humaine. Au sens de la présente méthode, le transcrit du gène DSG1 humain est le polypeptide dont la séquence a pour référence NCBI NM_001942.2. Par peptide précurseur de la protéine desmogléine 1 humaine, on entend ici le peptide dont la séquence peptidique est la séquence de référence NCBI : NP_001933.2.

Au sens de la présente méthode, le marqueur biologique involucrine comprend le gène IVL humain (référence NCBI : Gene ID: 3713), ainsi que les produits de ce gène. Les produits du gène IVL humain comprennent le transcrit du gène IVL humain et la protéine involucrine humaine. Au sens de la présente méthode, le transcrit du gène IVL humain est le polypeptide dont la séquence a pour référence NCBI NM_005547.2. Par protéine involucrine humaine, on entend ici la protéine dont la séquence peptidique est la séquence de référence NCBI : NP_005538.2.

Les inventeurs ont de plus montré que le perséose d'avocat entraine *in vitro,* une augmentation de l'expression des céramides 1 à 9, en particulier du céramide 1.

Les céramides sont les lipides majoritaires de la bicouche lamellaire et sont donc très importants pour la structure et fonctionnalité du *stratum corneum* en tant que barrière. Le céramide 1, en particulier, est sous-représenté dans les peaux sèches, âgées et aussi dans de nombreuses dermatoses comme la dermatite atopique, le psoriasis, l'ichtyose et l'acné.

La mesure de ces marqueurs chez le sujet traité par le perséose d'avocat offre donc un outil simple et solide pour mesurer l'efficacité de l'actif, et plus particulièrement l'efficacité de l'actif sur le maintien ou la restauration de la barrière lipidique.

Dans l'invention, le marqueur de la barrière lipidique est choisi parmi les céramides. Plus préférentiellement, le marqueur de la barrière lipidique est choisi parmi les céramides 1 à 9. Encore plus préférentiellement, le marqueur de la barrière lipidique est le céramide 1.

La protéine filaggrine s'agrège aux fibres de kératine du cytosquelette, réduisant ainsi les cornéocytes en disques aplatis, ce réseau intracellulaire confère résistance et protection au stratum corneum. D'autre part, sa dégradation conduit à la formation des constituants du NMF (Natural Moisturizing Factor ou Facteur Naturel d'Hydratation), qui est essentiel à la rétention d'eau dans les cornéocytes. La dégradation de la filaggrine est assurée par des peptidylarginine déiminases (PADI) dont PAD1 (Peptidyl Arginine Deiminase Type 1). L'acide hyaluronique est retrouvé dans l'épiderme où il joue un rôle dans la fonction barrière et l'hydratation de la couche cornée. L'enzyme transglutaminase 1 participe à la mise en place de l'enveloppe cornée en catalysant la réticulation de la protéine involucrine.

La mesure d'au moins un de ces marqueurs peut donc être utilisée pour estimer l'efficacité de l'actif sur la régulation hydrique de la couche cornée.

Dans l'invention, le marqueur de la régulation hydrique est choisi parmi la filaggrine, PAD1 (Peptidyl Arginine Deiminase Type 1), l'acide hyaluronique et la transglutaminase 1.

Au sens de la présente méthode, le marqueur biologique filaggrine comprend le gène FLG humain (référence NCBI : Gene ID: 2312), ainsi que les produits de ce gène. Les produits du gène FLG humain comprennent transcrit du gène FLG humain et la protéine filaggrine humaine. Au sens de la présente méthode, le transcrit du gène FLG humain est le polypeptide dont la séquence a pour référence NCBI NM_002016.1. Par protéine filaggrine humaine, on entend ici la protéine dont la séquence peptidique est la séquence de référence NCBI : NP_002007.1.

Au sens de la présente méthode, le marqueur biologique PAD1 comprend le gène PADI1 humain (référence NCBI : Gene ID: 29943 ainsi que les produits de ce gène. Les produits du gène PADI1 humain comprennent le transcrit du gène PADI1 humain et la protéine « Peptidyl Arginine Deiminase Type 1» humaine, ci-après appelée protéine PAD1 humaine. Au sens de la présente méthode, le transcrit du gène PADI1 humain est le polypeptide dont la séquence a pour référence NCBI NM_013358.2. Par protéine PAD1 humaine, on entend ici la protéine dont la séquence peptidique est la séquence de référence NCBI : NP_037490.2.

Au sens de la présente méthode, le marqueur biologique transglutaminase 1 comprend le gène TGM1 humain (référence NCBI : Gene ID: 7051), ainsi que les produits de ce gène. Les produits du gène TGM1 humain comprennent le transcrit du gène TGM1 humain et la protéine transglutaminase 1 humaine. Au sens de la présente méthode, le transcrit du gène TGM1 humain est le polypeptide dont la séquence a pour référence NCBI NM_000359.2. Par protéine transglutaminase 1 humaine, on entend ici la protéine dont la séquence peptidique est la séquence de référence NCBI : NP_000350.1.

En outre, il est connu que le *stratum granulosum* joue un rôle dans le bon fonctionnement du *stratum corneum,* et participe ainsi à la fonction barrière. Les inventeurs ont montré que, de façon surprenante, le perséose d'avocat, permet *in vitro* d'augmenter l'expression de la protéine claudine 1. La protéine claudine 1 est une protéine à 4 domaines transmembranaires appartenant à une famille de 24 membres. Elle est un constituant des jonctions serrées du *stratum granulosum,* qui forment une barrière sélective contrôlant le transport paracellulaire de molécules, de cellules inflammatoires. Ces jonctions serrées sont également essentielles pour limiter les pertes en eau.

Ainsi, en mesurant le niveau d'expression des marqueurs de la régulation du *stratum granulosum,* il est possible d'évaluer ou de vérifier l'efficacité du perséose d'avocat sur un sujet en particulier.

Dans l'invention, le marqueur de la régulation du *stratum granulosum* est choisi parmi les claudines. Plus préférentiellement, le marqueur de la régulation du *stratum granulosum* est la claudine 1.

Au sens de la présente méthode, le marqueur biologique claudine 1 comprend le CLDN1 humain (référence NCBI : Gene ID: 9076), ainsi que les produits de ce gène. Les produits du gène CLDN1 humain comprennent le transcrit du gène CLDN1 humain et la protéine claudine 1 humaine. Au sens de la présente méthode, le transcrit du gène CLDN1 humain est le polypeptide dont la séquence a pour référence NCBI NM_021101.4. Par protéine claudine 1 humaine, on entend ici la protéine dont la séquence peptidique est la séquence de référence NCBI : NP_066924.1.

L'homme du métier cherchant à déterminer à quelle classe appartient un marqueur génique ou protéique pourra facilement consulter la littérature scientifique pertinente ou se rapporter à des bases de données publiques telles que, par exemple, celles regroupées sur le site internet du National Center for Biotechnology Information (http://www.ncbi.nlm.nih.gov/guide/).

Par exemple, l'étape a) comprend la mesure du niveau d'expression et/ou d'activation d'une combinaison de marqueurs biologiques à partir d'un échantillon de cellules cutanées du sujet, caractérisé en ce que ladite combinaison de marqueurs comprend:
- au moins un marqueur de la maturation épidermique, ledit marqueur de la maturation épidermique étant de préférence choisi parmi la desmogléine 1 et l'involucrine ;
- au moins un marqueur de la barrière lipidique, ledit marqueur de la barrière lipidique étant de préférence choisi parmi les céramides, en particulier parmi les céramides 1 à 9, encore plus particulièrement le céramide 1 ; et
- au moins un marqueur de la régulation hydrique, ledit marqueur de la régulation hydrique étant de préférence choisi parmi la filaggrine, PAD1, l'acide hyaluronique et la transglutaminase 1 ;
- au moins un marqueur de la régulation du *stratum granulosum,* ledit marqueur de la régulation du *stratum granulosum* étant de préférence choisi parmi les claudines, en particulier la claudine 1

Préférentiellement, la combinaison de marqueurs biologiques consiste en la desmogléine 1, l'involucrine, le céramide 1, la filaggrine, PAD1, l'acide hyaluronique, la transglutaminase 1 et la claudine 1.

Lorsque le marqueur biologique est un gène, les termes «niveau d'expression du marqueur biologique » se réfèrent au niveau de synthèse des produits de ce gène. Plus précisément, lorsque le marqueur biologique est un gène, le niveau d'expression dudit marqueur biologique correspond à la quantité ou à la concentration des transcrits dudit gène et/ou des différents isoformes des protéines issus desdits transcrits dans l'échantillon.

Lorsque le marqueur biologique est un lipide, un sucre ou un métabolite, les termes «niveau d'expression du marqueur biologique » se réfèrent au niveau de synthèse dudit lipide, dudit sucre ou dudit métabolite. Plus précisément, lorsque le marqueur biologique est un lipide, un sucre ou un métabolite, le niveau d'expression dudit marqueur biologique correspond à la quantité ou à la concentration dudit lipide, dudit sucre ou dudit métabolite dans l'échantillon.

Les termes « niveau d'activation du marqueur biologique » s'appliquent aux marqueurs biologiques pour lesquels une activité biologique, en particulier une activité enzymatique, peut être mesurée. L'homme du métier sait par exemple que certains peptides présentent une activité enzymatique. L'homme du métier cherchant à déterminer si un marqueur biologique d'intérêt présente une activité, en particulier enzymatique, pourra facilement consulter la littérature scientifique pertinente ou se rapporter à des bases de données publiques telles que, par exemple, celles regroupées sur le site internet ExPASy, qui répertorie notamment les enzymes (http://enzyme.expasy.org). Selon la présente méthode, les marqueurs PAD1 et transglutaminase 1 sont des marqueurs biologiques pour lesquels une activité enzymatique peut être mesurée.

Lorsque le marqueur biologique est un marqueur biologique pour lequel une activité enzymatique peut être mesurée, les termes « niveau d'activation du marqueur biologique » se réfèrent niveau d'activité enzymatique dudit marqueur. Au sens de la présente méthode, l'activité enzymatique dudit marqueur correspond à la quantité de substrat dudit marqueur catalysée par unité de temps grâce à un quantum défini d'enzyme. L'unité d'activité enzymatique U est classiquement exprimée en µmol/min ou en kat (mol/s). L'unité d'activité enzymatique U peut aussi être exprimée en mg/min lorsque le substrat de l'enzyme existe dans le corps sous forme de polymère, tel que c'est le cas par exemple lorsque le substrat est choisi parmi les sucres ou les glycosaminoglucanes.

Par la « mesure du niveau d'expression et/ou d'activation d'une combinaison de marqueurs biologiques », on entend ici la mesure du niveau d'expression et/ou d'activation de chacun des marqueurs de la combinaison.

L'expression d'un gène peut être mesurée par exemple au niveau nucléotidique, en mesurant la quantité de transcrits dudit gène et leurs variants, et peut aussi être mesurée par exemple au niveau protéique, en mesurant par exemple la quantité d'un ou plusieurs isoformes des protéines issus desdits transcrits, et leurs variants. Ainsi, par « mesure du niveau d'expression dudit gène » on entend ici la mesure de la quantité de produit du gène sous sa forme peptidique et/ou sous sa forme nucléotidique.

Par « desmogléine 1 sous sa forme peptidique », on entend ici un peptide dont la séquence peptidique comprend la séquence peptidique de la protéine desmogléine 1 humaine (dont la séquence codante est référencée CCDS11896.1), et/ou son peptide précurseur. Par « peptide précurseur de la protéine desmogléine 1 humaine », on entend le prepro-desmogléine 1 humaine (référence NCBI : NP_001933.2).

Par « desmogléine 1 sous sa forme nucléotidique », on entend ici au moins un polynucléotide dont la séquence comprend la séquence du transcrit du gène DSG1 humain (référence NCBI : GENE ID 1828). Au sens de la présente invention, le transcrit du gène DSG1 humain est le polypeptide dont la séquence a pour référence NCBI NM_001942.2.

Par « involucrine sous sa forme peptidique », on entend ici un peptide dont la séquence peptidique comprend la séquence peptidique de la protéine involucrine humaine (référence NCBI : NP_005538.2).

Par « involucrine sous sa forme nucléotidique », on entend ici au moins un polynucléotide dont la séquence comprend la séquence du transcrit du gène IVL humain (référence NCBI : Gene ID: 3713). Au sens de la présente méthode, le transcrit du gène IVL humain est le polypeptide dont la séquence a pour référence NCBI NM_005547.2.

Par « filaggrine sous sa forme peptidique », on entend ici un peptide dont la séquence peptidique comprend la séquence peptidique de la protéine filaggrine humaine (référence NCBI : NP_002007.1).

Par « filaggrine sous sa forme nucléotidique », on entend ici au moins un polynucléotide dont la séquence comprend la séquence du transcrit du gène FLG humain (référence NCBI : Gene ID: 2312). Au sens de la présente méthode, le transcrit du gène FLG humain est le polypeptide dont la séquence a pour référence NCBI NM_002016.1.

Par « PAD1 sous sa forme peptidique », on entend ici un peptide dont la séquence peptidique comprend la séquence peptidique de la protéine humaine (référence NP_037490.2).

Par « PAD1 sous sa forme nucléotidique », on entend ici au moins un polynucléotide dont la séquence comprend la séquence du transcrit du gène PADI1 humain (référence NCBI : Gene ID: 29943). Au sens de la présente méthode, le transcrit du gène PADI1 humain est le polypeptide dont la séquence a pour référence NCBI NM_013358.2.

Par « transglutaminase 1 sous sa forme peptidique », on entend ici un peptide dont la séquence peptidique comprend la séquence peptidique de la protéine transglutaminase 1 humaine (référence NCBI : NP_000350.1).

Par « transglutaminase 1 sous sa forme nucléotidique », on entend ici au moins un polynucléotide dont la séquence comprend la séquence du transcrit du gène TGM1 humain (référence NCBI : Gene ID: 7051). Au sens de la présente méthode, le transcrit du gène TGM1 humain est le polypeptide dont la séquence a pour référence NCBI NM_000359.2.

Par « claudine 1 sous sa forme peptidique », on entend ici un peptide dont la séquence peptidique comprend la séquence peptidique de la protéine claudine 1 humaine (référence NCBI : NP_066924.1).

Par « claudine 1 sous sa forme nucléotidique », on entend ici au moins un polynucléotide dont la séquence comprend la séquence du transcrit du gène CLDN1 humain (référence NCBI : Gene ID: 9076). Au sens de la présente méthode, le transcrit du gène CLDN1 humain est le polypeptide dont la séquence a pour référence NCBI NM_021101.4.

De préférence, le niveau d'expression du marqueur choisi parmi la desmogléine 1, de l'involucrine, ou de la filaggrine correspond au niveau d'expression dudit marqueur sous sa forme peptidique.

De préférence, le niveau d'expression de PAD1 correspond au niveau d'expression dudit marqueur sous sa forme nucléotidique.

De façon générale, la mesure du niveau d'expression et/ou d'activation du marqueur biologique sera détectée *in vitro* à partir d'un échantillon de cellules cutanées du sujet.

Par « échantillon de cellules cutanées du sujet », on entend ici tout échantillon contenant des cellules de la peau obtenu du sujet. Les échantillons de cellules cutanées selon la méthode comprennent donc aussi bien les explants de peau frais obtenus directement du sujet, que les cultures de cellules cutanées en suspension, les cultures de cellules cutanées en monocouche, les cultures de cellules cutanées en bicouche et les modèles tissulaires, dont les cultures de peaux reconstruites et les cultures de muqueuses reconstruites. Comme il est souvent difficile de travailler sur des explants frais, il est particulièrement avantageux, dans le cadre de la présente méthode, d'utiliser des cultures de cellules cutanées. Avantageusement, les cellules cutanées selon la méthode comprennent des cellules normales, saines ou pathologiques, ou des cellules issues de lignées. Par exemple, les cellules cutanées mises en culture peuvent être des cellules obtenues à partir d'explant de tissu cutané. Par « explant » ou « explant de peau », on entend ici un prélèvement de cellules ou de tissu cutané, lequel peut être réalisé dans un but chirurgical ou pour effectuer des analyses.

En particulier, un explant peut avoir été obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé ; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés dans la présente méthode.

Les cellules cutanées selon la méthode comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées selon la méthode comprennent au moins des kératinocytes et/ou des fibroblastes. De façon plus préférentielle, les cellules cutanées comprennent des kératinocytes et/ou des fibroblastes. Il peut être intéressant pour l'homme du métier d'évaluer l'efficacité des actifs sur des cellules du sujet soumises à certains stress, tels que par exemple les parties de la peau du sujet soumises aux expositions solaires, ou de façon plus générale, aux irradiations aux UV. Par exemple, l'échantillon de cellules cutanées comprend des cellules ayant été irradiées, préférentiellement par UV.

Par exemple, l'échantillon de cellules cutanées peut être traité préalablement à la mesure de l'expression du marqueur biologique, par exemple afin d'extraire à partir dudit échantillon de cellules cutanées un échantillon d'ARNm ou un échantillon de protéine. L'échantillon d'ARNm ou de protéine peut ensuite être directement utilisé pour mesurer l'expression du marqueur. La préparation ou l'extraction d'ARNm ou de protéines à partir d'un échantillon cellulaire ou tissulaire sont des procédures de routine bien connues de l'homme du métier.

Pour chacun des types de marqueurs biologiques de la méthode, de nombreuses méthodes sont à la disposition de l'homme du métier pour mesurer le niveau d'expression et/ou d'activation dudit marqueur biologique.

Quand le marqueur biologique est un gène, et que le niveau d'expression du marqueur est mesuré au niveau nucléotidique, c'est-à-dire en mesurant la quantité de produit du gène sous sa forme nucléotidique, n'importe quelle technologie habituellement utilisée par l'homme du métier peut être mise en oeuvre. Les méthodes d'analyse du niveau d'expression des gènes au niveau nucléotidique, comme par exemple l'analyse du transcriptome, incluent des méthodes bien connues telles que la RT-PCR ou la RT-PCR quantitative ou encore les puces d'acides nucléiques.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose. Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides. Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Préférentiellement, la méthode est mise en oeuvre en utilisant toute méthode actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente *in situ.* Pour l'analyse au niveau de l'ARNm, les puces à tissu peuvent être couplées avec l'hybridation fluorescente in situ. Enfin, il est possible d'utiliser le séquençage massif en parallèle pour déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127, U.S. 4,849,077; U.S. 7,556,922; U.S. 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45. 2008; Pihlak et al., Nat Biotechnol., 26(6) : 676- 684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

Quand le marqueur biologique est un gène, et que le niveau d'expression du marqueur est mesuré au niveau peptidique, c'est-à-dire en mesurant la quantité de produit du gène sous sa forme peptidique, toute méthode pour déterminer le niveau d'expression d'un polypeptide connue de l'homme du métier peut être utilisée. Les méthodes pour déterminer le niveau d'expression d'un polypeptide incluent par exemple la spectrométrie de masse, les tests biochimiques, y compris les tests immunologiques tels que par exemple les tests immunologiques de détection classiques (les tests ELISA et les tests ELISPOTS), ou encore comme par exemple des tests immunologiques employant des techniques de transfert des protéines sur support, tels que le slot blot (aussi appelé dot blot) ou le western blot. Il est par exemple possible d'employer les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

De préférence, le niveau d'expression du marqueur choisi parmi la desmogléine 1, l'involucrine, ou la filaggrine sous sa forme peptidique est mesuré par ELISA. Un exemple d'anticorps utilisable pour un test ELISA adapté à la mesure du niveau d'expression de la desmogléine 1 au niveau peptidique est l'anticorps polyclonal de lapin contre la desmogéine commercialisé par Abcam (ref ab14417). Un exemple d'anticorps utilisable pour un test ELISA adapté à la mesure du niveau d'expression de l'involucrine au niveau peptidique est l'anticorps monoclonal de souris contre l'involucrine commercialisé par Sigma (ref 19018). Un exemple d'anticorps utilisable pour un test ELISA adapté à la mesure du niveau d'expression de la filaggrine au niveau peptidique est l'anticorps polyclonal de lapin contre la filaggrine commercialisé par Abcam (ref ab81468).

Lorsque le marqueur biologique est un lipide, un sucre ou un métabolite, l'homme du métier pourra utiliser toute méthode de mesure permettant le dosage dudit marqueur dans l'échantillon du sujet. Ces méthodes incluent par exemple la spectrométrie de masse et les tests biochimiques. De préférence, le niveau d'expression de de l'acide hyaluronique est mesuré par test ELISA en compétition. Le test ELISA en compétition de l'acide hyaluronique est un dosage compétitif qui utilise une protéine se liant spécifiquement à l'acide hyaluronique marquée (par exemple biotinylée), c'est-à-dire capable de se lier à l'acide hyaluronique avec une grande affinité, et des microplaques recouvertes d'acide hyaluronique. La protéine de liaison marquée se fixe sur l'acide hyaluronique immobilisé, si celui-ci n'est pas saturé par l'acide hyaluronique libre contenu dans l'échantillon. Lors de l'incubation suivante, un conjugué capable de se lier au marqueur de la protéine marquée (par exemple un conjugué couplé à la Streptavidine péroxydase) se fixe sur la protéine de liaison marquée. On mesure la réaction du substrat par l'intensité de la couleur, qui est inversement proportionnelle au taux d'acide hyaluronique contenu dans les échantillons. Un exemple de protéine se liant spécifiquement à l'acide hyaluronique (« HA detector ») et d'anticorps utilisables pour un test ELISA en compétition adapté à la mesure du niveau d'expression de l'acide hyaluronique sont ceux du kit Tebu (ref K-1200-0001). Les lipides, en particulier les céramides, pourront par exemple être dosés par des méthodes classiques de mesure d'incorporation d'acétate radiomarqué dans les kératinocytes, ou épidermes reconstruits, ou explants de peau.

Par « échantillon de cellules cutanées de référence », on entend ici tout échantillon contenant des cellules de la peau humaines utilisé à titre d'échantillon de référence. L'homme du métier saura choisir l'échantillon de cellules cutanées de référence en fonction par exemple de l'âge, du sexe, de la couleur de peau du sujet testé.

De préférence, l'échantillon de cellules cutanées de référence est un échantillon de cellules cutanées d'un enfant. De façon particulièrement préféré, l'échantillon de cellules cutanées de référence est un échantillon de cellules cutanées d'un enfant de 2 ans ou plus, d'un nourrisson ou d'un nouveau-né.

L'homme du métier pourra en outre choisir comme échantillon de cellules cutanées de référence un échantillon de cellules n'ayant pas été traité, ou ayant été irradié, par exemple par des UV. Préférentiellement, lorsque le marqueur biologique selon la méthode est la claudine 1, ou lorsque la combinaison de marqueurs biologiques de la méthode comprend la claudine 1, l'échantillon de cellules cutanées de référence est un échantillon de cellules ayant été irradiées par des UV.

En particulier, l'échantillon de cellules cutanées de référence selon la méthode est un échantillon de cellules cutanées dudit sujet n'ayant pas été traitées par ledit actif.

Alternativement, en particulier, l'échantillon de cellules cutanées de référence selon la méthode est un échantillon de cellules cutanées d'un sujet présentant ladite déficience de la barrière cutanée, ledit sujet n'ayant pas été traité pour ladite déficience.

En particulier, l'échantillon de cellules cutanées de référence selon la méthode est un échantillon de cellules cutanées d'un sujet sain.

L'homme du métier comprendra aisément que la comparaison de l'étape c) est de préférence effectuée entre des mesures de niveaux d'expression obtenus pour des échantillon de cellules cutanées qui sont de taille, de volume ou de poids similaire. Ainsi, il est préférable que la taille, et/ou le volume et/ou le poids de l'échantillon de l'étape a) ne diffère pas de plus de 5% de la taille, et/ou le volume et/ou du poids de l'échantillon de l'étape b). Encore plus préférentiellement, la taille, et le volume et le poids de l'échantillon de l'étape a) ne diffère pas de plus de 5% de la taille, du volume du poids de l'échantillon de l'étape b).

Alternativement, si les échantillons des étapes a) et b) diffèrent de plus de 5 % de par la taille, et/ou le volume et/ou le poids, l'homme du métier pourra normaliser les niveaux obtenus aux étapes a) et b) à l'aide d'un facteur de normalisation. Ce facteur pourra par exemple être un marqueur physique directement accessible tel que la masse de cellules de l'échantillon, ou la masse d'un constituant cellulaire, comme la masse d'ADN cellulaire ou la masse de protéines cellulaires.

Il peut aussi être avantageux d'utiliser comme facteur de normalisation le niveau d'expression d'un gène qui est exprimé au même niveau dans toutes les cellules, ou presque, de l'organisme. En d'autres termes, en particulier, on utilise comme facteur de normalisation le niveau d'expression d'un gène de ménage. Par exemple, les niveaux des étapes a) et b) sont normalisés en utilisant le niveau d'expression, non pas des gènes de ménage, mais des protéines codées par ceux-ci. Un gène de ménage est un gène exprimé dans tous les types cellulaires, qui code une protéine ayant une fonction de base nécessaire pour la survie de tous les types cellulaires. Une liste de gènes de ménage humains peut être trouvée dans Eisenberg et al. (Trends in Genetics 19: 362-365, 2003). Les gènes de ménage selon la méthode incluent par exemple les gènes B2M, TFRC, YWHAZ, RPLO, 18S, GUSB, UBC, TBP, GAPDH, PPIA, POLR2A, ACTB, PGK1, HPRT1, IPO8 et HMBS.

L'homme du métier pourra ainsi aisément évaluer de l'efficacité de l'actif de la méthode en fonction de la comparaison de l'étape c).

Par exemple, lorsque le niveau de desmogléine 1, d'involucrine, de filaggrine, de claudine 1 et/ou ou d'acide hyaluronique, en particulier sous leur forme peptidique, mesuré à l'étape a), est supérieur ou égal au niveau mesuré à l'étape b) dans laquelle ledit échantillon de cellules cutanées de référence est un échantillon de cellules cutanées d'un sujet sain, de préférence un échantillon de cellules cutanées d'un enfant, d'un enfant de 2 ans ou plus, d'un nourrisson ou d'un nouveau-né, alors l'actif est efficace dans la prévention et/ou le traitement d'au moins une déficience de la barrière cutanée d'un sujet.

De même, par exemple, lorsque le niveau de desmogléine 1, d'involucrine, de céramide 1, de filaggrine, de PAD1, d'acide hyaluronique, de transglutaminase 1 et/ou de claudine 1, mesuré à l'étape a), est supérieur au niveau du même marqueur mesuré à l'étape b) dans laquelle ledit échantillon de cellules cutanées de référence est un échantillon de cellules cutanées dudit sujet n'ayant pas été traitées par ledit actif, ou d'un sujet présentant ladite déficience de la barrière cutanée et n'ayant pas été traité pour ladite déficience, alors l'actif est efficace dans la prévention et/ou le traitement d'au moins une déficience de la barrière cutanée d'un sujet.

Par exemple, lorsque le niveau de desmogléine 1, d'involucrine, de céramide 1, de filaggrine, de PAD1, d'acide hyaluronique, de transglutaminase 1 et/ou de claudine 1 mesuré à l'étape a), est inférieur ou égal au niveau du même marqueur mesuré à l'étape b) dans laquelle ledit échantillon de cellules cutanées de référence est un échantillon de cellules cutanées dudit sujet n'ayant pas été traitées par ledit actif, ou d'un sujet présentant ladite déficience de la barrière cutanée et n'ayant pas été traité pour ladite déficience, alors l'actif n'est pas efficace dans la prévention et/ou le traitement d'au moins une déficience de la barrière cutanée d'un sujet. Par « déficience de la barrière cutanée », on entend au sens de la présente demande une ou des modifications physiologiques ou pathologiques de la barrière cutanée, plus particulièrement une ou des modifications physiologiques ou pathologiques de la structure et/ou de la fonction du *stratum corneum* et /ou du *stratum granulosum,* en comparaison avec une peau normale de sujet adulte.

Au sens de la présente méthode, la déficience de la barrière cutanée peut être due à une modification physiologique de la barrière cutanée.

Les peaux des enfants, par exemple, même lorsqu'elles sont saines, peuvent présenter une barrière qui n'est pas pleinement fonctionnelle en regard de la peau d'adulte. En effet, la maturation de la peau d'enfant s'accompagne d'un développement de la fonction barrière de celle-ci. De même, les peaux des sujets âgés présentent elles aussi des modifications de la barrière cutanée, en comparaison avec la peau normale des adultes, du fait de leur sénescence. Au sens de la présente méthode, les peaux des enfants et les peaux des sujets âgés présentent au moins une déficience de la barrière cutanée. L'immaturité de la peau des enfants, et la sénescence de la peau du sujet âgé sont considérées comme des déficiences de la barrière cutanée au sens de la présente méthode.

En outre, la déficience de la barrière cutanée peut être due à une modification pathologique de la barrière cutanée.

Par exemple, les peaux endommagées par des facteurs externes tels que les conditions climatiques, la pollution, les blessures (par exemple les coupures ou les brulures), les irritations par agent chimique présentent des diminutions de la barrière cutanées.

Par ailleurs, certaines pathologies dermatologiques sont associées à des affaiblissements de la barrière cutanée, telles que par exemple l'acné du nourrisson, l'acné de l'adolescent, la rosacée ou érythrocouperose, le psoriasis, la dermite du siège, la dermatite atopique, l'eczéma, la dermatite de contact, la dermatite irritative et en particulier la dermatite irritative du siège ou érythème fessier, la dermatite allergique, la dermite séborrhéique, la sécheresse cutanée, l'hyperréactivité cutanée.

Préférentiellement, la déficience de la barrière cutanée est choisie parmi l'acné du nourrisson, l'acné de l'adolescent, la rosacée ou érythrocouperose, le psoriasis, la dermite du siège, la dermatite atopique, l'eczéma, la dermatite de contact, la dermatite irritative et en particulier la dermatite irritative du siège ou érythème fessier, la dermatite allergique, la dermite séborrhéique, la peau sensible, la peau réactive, la xérose, la peau déshydratée, la peau endommagée par le soleil, par les radiations, par le vent, par le froid, par le chaud, par le stress, par la pollution, l'érythème cutané, la peau âgée ou photo âgée, la peau photosensibilité, les dartres, les ichtyoses, les gerçures, les brûlures, les coups de soleil, les inflammations dus aux rayons de toutes sortes, les irritations par agents chimiques, physiques, bactériologiques, fongiques ou viraux, parasitaires.

La méthode permet d'évaluer l'efficacité d'un actif choisi parmi les sucres en C7 et les dérivés de formule (I), 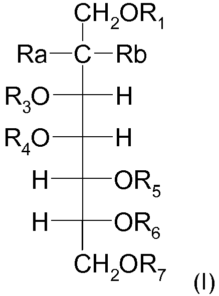 où
Ra représente un atome d'hydrogène et Rb représente un -OR2 ou CRaRb représente le radical CO ;
R1, R2, R3, R4, R5, R6 et R7 représentent, indépendamment l'un de l'autre
   - un atome d'hydrogène ou
   - un radical -(CO)-R dans lequel R représente une chaîne hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique ; ou
   - un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique.

En particulier, l'actif est le D-mannoheptulose, de formule générale (II)

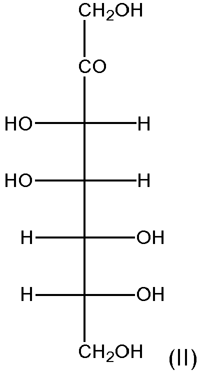

En particulier, l'actif est le perséitol, de formule générale (III)

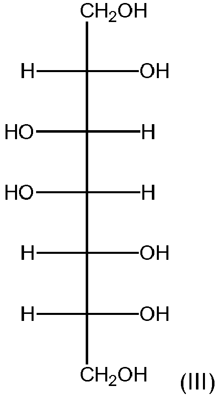

La source de sucres en C7, en particulier de D-mannoheptulose et/ou de perséitol, peut être un extrait hydrosoluble de sucres d'avocat ou d'une autre plante. Autrement, le D-mannoheptulose et le perséitol sont disponibles commercialement (origine synthétique). Selon une variante avantageuse de la méthode, la source de D-mannoheptulose et/ou de perséitol est un extrait hydrosoluble de sucres d'avocat.

L'extrait hydrosoluble de sucres d'avocat peut directement être obtenu à partir de n'importe quelle partie de l'avocat ou de l'avocatier, telle que le fruit, la peau, le noyau, la feuille ou les racines de l'avocatier. Il est aussi possible d'obtenir un extrait hydrosoluble de sucres à partir des co-produits de l'industrie de transformation de l'avocat, parmi lesquels on peut citer de façon non exhaustive : la pulpe fraîche d'avocat, la pulpe congelée, déshydratée, les tourteaux d'avocat issus des procédés d'extraction d'huile (extraction mécanique et/ou par solvant du fruit préalablement déshydraté), les matières solides déshuilées issues des procédés d'extraction d'huile par voie humide (procédé dit de centrifugation), les matières solides déshuilées issues des procédés d'extraction d'huile d'avocat par voie enzymatique, les purées d'avocat brutes (guacamole), les déchets solides issus des unités de production de ces purées. L'extrait est avantageusement obtenu à partir du fruit frais de l'avocatier. Les fruits pourront être choisis parmi les variétés Hass, Fuerte, Ettinger, Bacon, Nabal, Anaheim, Lula, Reed, Zutano, Queen, Criola Selva, Mexicana Canta, Région Dschang, Hall, Booth, Peterson, Collinson Redn, plus avantageusement parmi les variétés Hass, Fuerte et Reed. De préférence, on retiendra les variétés Hass, Fuerte, Ettinger et Bacon, et plus avantageusement les variétés Hass et Fuerte.

Afin d'obtenir les sucres en C7, en particulier de D-mannoheptulose et/ou de perséitol, toute méthode permettant d'obtenir des extraits osidiques de plantes connues de l'homme du métier pourra être utilisée. L'homme du métier pourra en particulier se reporter au procédé décrit dans la demande FR 2 843 027.

Par exemple, les sucres en C7, en particulier le D-mannoheptulose et le perséitol, sont au moins partiellement estérifiés avec un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique. En particulier les sucres en C7 sont au moins partiellement estérifiés avec un résidu d'acide gras. La chaine hydrocarbonée peut être linéaire ou ramifiée, elle est avantageusement linéaire.

Il sera évident pour l'homme du métier que les actifs peuvent être formulés, afin d'en faciliter l'administration par exemple. Par exemple, l'actif est formulé sous la forme d'une composition, préférentiellement adaptée à une administration topique.

La composition peut en outre comprendre au moins un autre composé actif en plus des sucres en C7 et des dérivés de formule (I). Cet autre composé peut être choisi parmi tous les composés et leurs équivalents fonctionnels, énoncés ci-dessous :
Cet autre composé peut être en particulier choisi parmi des actifs classiquement utilisés en dermatologie ou cosmétique tels que les émollients, les actifs hydratants, les activateurs de la synthèse de kératine, les kératorégulateurs, les kératolytiques, les agents restructurant de la barrière cutanée, des agonistes PPARs (ou Peroxysome Proliferator Activated Receptor), les agonistes RXR ou LXR, les agents cicatrisants, les agents sébo-régulateurs, les agents anti-irritants, les agents apaisants, les agents anti-inflammatoires, les agents anti-oxydants et les agents anti-âge, les agents dépigmentants ou hypodépigmentants, les agents pigmentants, les agents lipolytiques ou inhibiteurs de la lipogénèse ou encore les agents anti-cellulite ou amincissants, les filtres et écrans solaires minéraux ou organiques, les composés antifongiques, les conservateurs, les agents anti-bactériens, les pré et probiotiques, les antibiotiques, les immuno-modulateurs.

La méthode est particulièrement utile pour suivre l'évolution des sujets, en fonction du traitement administré et peut donc être utilisée par le praticien pour choisir au mieux le traitement à administrer au sujet.

Il est également ici décrit une méthode pour adapter le traitement d'un sujet, ledit traitement comprenant un actif choisi parmi les sucres en C7 et les dérivés de formule (I), 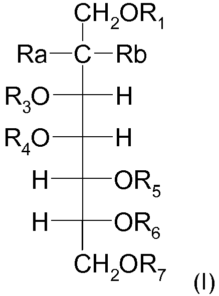 où
Ra représente un atome d'hydrogène et Rb représente un -OR2 ou CRaRb représente le radical CO ;
R1, R2, R3, R4, R5, R6 et R7 représentent, indépendamment l'un de l'autre
   - un atome d'hydrogène ou
   - un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique ; ou
   - un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique ;
ladite méthode comprenant les étapes
a) d'évaluation de l'efficacité dudit actif par une méthode pour évaluer l'efficacité d'un actif;
b) d'adaptation dudit traitement en fonction des résultats obtenus à l'étape a).

L'homme du métier comprendra aisément que la méthode pour adapter le traitement d'un sujet peut être mise en oeuvre pour les divers actifs mentionnés plus haut, ainsi que pour les compositions les comprenant.

En particulier, l'actif est le D-mannoheptulose, de formule générale (II)

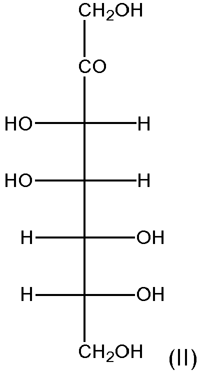

En particulier, l'actif est le perséitol, de formule générale (III)

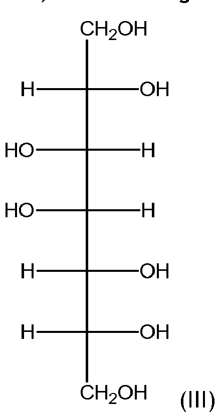

L'homme du métier pourra aisément adapter ledit traitement en fonction des résultats obtenus à l'étape a).

Par exemple, si l'évaluation de l'étape a) montre que l'actif n'est pas efficace dans le traitement et/ou la prévention d'au moins une déficience de la barrière, l'homme du métier pourra en conséquence augmenter les quantités d'actif administré, la concentration en actif ou encore la fréquence des administrations. Alternativement, l'homme du métier pourra par exemple adapter le traitement en ajoutant d'autres actifs complémentaires du perséose d'avocat.

Si, en revanche, l'actif est efficace, tel que déterminé à l'étape a, dans le traitement et/ou la prévention d'au moins une déficience de la barrière, l'homme du métier pourra par exemple continuer le traitement sans modifier les quantités d'actif administré, la concentration en actif ou encore la fréquence des administrations, diminuer les quantités d'actif administré, la concentration en actif ou encore la fréquence des administrations, ou encore discontinuer le traitement. La méthode décrite ici constitue donc un outil pratique et une aide à la décision pour l'homme du métier.

Il est ici décritun kit pour la mise en oeuvre d'une méthode décrite ci-dessus, comprenant les moyens nécessaires à la mesure du niveau d'expression et/ou d'activation d'au moins un marqueur biologique choisi parmi la desmogléine 1, l'involucrine, les céramides, en particulier parmi les céramides 1 à 9, encore plus particulièrement le céramide 1, la filaggrine, PAD1, l'acide hyaluronique, la transglutaminase 1, et les claudines, en particulier la claudine 1.

En particulier, le kit comprend les moyens nécessaires à la mesure du niveau d'expression et/ou d'activation de chacun des marqueurs biologiques de la combinaison consistant en la desmogléine 1, l'involucrine, le céramide 1, la filaggrine, PAD1, l'acide hyaluronique, la transglutaminase 1 et la claudine 1. Préférentiellement, les moyens nécessaires à la mesure du niveau d'expression de la desmogléine 1, de l'involucrine, de la filaggrine, de la claudine 1 ou de l'acide hyaluronique sont des anticorps spécifiques desdits marqueurs. Préférentiellement, les moyens nécessaires à la mesure du niveau d'expression de PAD1 comprennent des sondes nucléiques et/ou des amorces d'amplification capables de se lier à PAD1 sous sa forme nucléotidique.

### Légendes des figures

Figure 1 : Niveaux d'expression du marqueur involucrine dans des kératinocytes humains normaux, avec ou sans traitement par du perséose d'avocat (perséose).
Figure 2 : Niveaux d'expression du marqueur desmogléine dans des kératinocytes humains normaux, avec ou sans traitement par du perséose d'avocat (perséose).
Figure 3 : Niveaux d'activité du marqueur transglutaminase dans des kératinocytes humains normaux, avec ou sans traitement par du perséose d'avocat.
Figure 4 : Niveaux d'expression du marqueur filaggrine dans des kératinocytes humains normaux, avec ou sans traitement par du perséose d'avocat.
Figure 5 : Niveaux d'expression du marqueur PAD1 dans des kératinocytes humains normaux, avec ou sans traitement par du perséose d'avocat.
Figure 6 : Niveaux d'expression du marqueur acide hyaluronique dans des kératinocytes humains normaux, avec ou sans traitement par du perséose d'avocat.
Les exemples qui suivent illustrent les méthodes et kits décrits ci-dessus mais ne sont pas limitatifs.

### Exemples

### 1. Exemple 1 : préparation d'un extrait hydrosoluble de sucres d'avocat

Les avocats frais, de la variété Hass, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 80° C, sa durée est quant à elle de 48 heures. Une fois séchés, les fruits sont soumis à une pression à froid. Cette opération est réalisée sur une petite presse Komet de laboratoire. Ainsi sont obtenus de l'huile et un tourteau.

Le tourteau est alors broyé puis extrait, en présence d'éthanol à 70 % ou d'eau. Les parties liquides et solides sont séparées par centrifugation par exemple. La fraction soluble (liquide) est reprise pour être purifiée et concentrée selon le mode opératoire suivant :
- Déminéralisation à l'aide de résines échangeuses d'ions : déminéralisation des heptuloses par passage sur résines OH-, puis sur résine H+.
- Ultrafiltration sur 10 000 Da : l'ultrafiltration est réalisée avec un système équipé de 4 membranes de seuil de coupure 10 kDa.
- Concentration sous vide : la concentration de l'extrait purifié est réalisée à l'aide d'un évaporateur sous vide jusqu'à l'obtention d'une matière sèche voisine de 4 %.
- Conditionnement : la concentration de l'extrait est ajustée à 5 % de matière sèche et on ajoute du conservateur, puis on filtre stérilement avec une membrane de 0,2 µm de seuil de coupure et on conditionne.

Le tableau 1 donne la composition de l'extrait de sucres d'avocat en C7, à 5 % de matière sèche, préparé suivant le procédé décrit ci-dessus :

**Tableau 1**

| Aspect | Solution de couleur jaune pâle |
|---|---|
| Critères analytiques | |
| Matière sèche | 5 % |
| pH (dilution ¼) | 7,0 |
| | |

| Composition (%/matière sèche) | |
|---|---|
| Saccharose | 3,0 |
| Glucose | 7,5 |
| D-mannoheptulose | 40,0 |
| Fructose | 8,6 |
| Perséitol | 40,0 |

Suivant ce même procédé, on a préparé deux autres extraits, dont la valeur du pH, l'absorbance et la teneur en sucres en C7 sont données dans le tableau 2. La teneur en sucres en C7 correspond à la somme du perséitol et du D-mannoheptulose analysée par HPLC.

**Tableau 2**

| Lot | 1 | 2 |
|---|---|---|
| Matière sèche | 5% | 5% |
| pH (dilution ¼) | 5,9 | 5,4 |
| Sucres en C7 / matière sèche | 80,5 | 83,4 |

Les sucres d'avocat préparés par le procédé de l'exemple 1 et appelés « perséose d'avocat » ci-dessous peuvent être utilisés dans différentes formulations.

### Exemples de formulations

### Crème hydratante

**Tableau 3**

| Matière première / Nom commercial | % |
|---|---|
| CAPRYLO CAPRATE GLYC | 1 à 15% |
| HUILE DE TOURNESOLSR | 1 à 15% |
| ALCOOL CETYLIQUE PUR | 1 à 5% |
| GLYCERYL STEARATE CITRATE | 1 à 10% |
| CIRE ABEILLE | 1 à 5% |
| EUMULGINSG | 0 à 2% |
| ACETATE VITAMINE E | 0 à 1% |
| EAU PURIFIEE | QSP 100% |
| CARBOPOL ULTREZ20 | 0 à 1% |
| GLYCEROL | 1 à 10% |
| GOMME XANTHANE | 0 à 1% |
| LESSIVE SOUDEXI | 0 à 1% |
| CONSERVATEUR | 0 à 2% |
| PERSEOSE AVOCAT | 0 à 1% |

### Lait restructurant

**Tableau 4**

| Matière première / Nom | % |
|---|---|
| EAU PURIFIEE | QSP 100% |
| HUILE DE TOURNESOLSR | 1 à 10% |
| HUILE DE COPRAH HYDRO | 1 à 10% |
| HUILE D'AMANDE DOUCE | 1 à 10% |
| HUILE DE MAIS | 1 à 10% |
| MONOSTEARATE GLYCEROL | 1 à 10% |
| ACIDE STEARIQUE | 1 à 10% |
| CONSERVATEUR | 0 à 2% |
| ALCOOL CETYLIQUE C16-C18 | 0 à 2% |
| ACETATE VITAMINE E | 0 à 1% |
| LESSIVE SOUDEXI | 0 à 1% |
| PERSEOSE AVOCAT | 0 à 1% |

### Eau nettoyante

**Tableau 5**

| Matière première / Nom | % |
|---|---|
| EAU PURIFIEE | QSP 100% |
| GLYCEROL | 1 à 10% |
| SODIUM COCOYL | 1 à 10% |
| CONSERVATEUR | 0 à 2% |
| RICIN HYDROBUTETH 26 | 0 à 2% |
| ALLANTOINE | 0 à 2% |
| ACIDE TARTRIQUE | 0 à 2% |
| ALOE VERA POUDRESR | 0 à 2% |
| EXTRAIT SAPONAIRESR | 0 à 2% |
| LESSIVE SOUDEXI | 0 à 2% |
| PERSEOSE AVOCAT | 0 à 1% |

### Shampooing

**Tableau 6**

| Matière première / Nom commercial | de |
|---|---|
| EAU PURIFIEE | QSP 100% |
| COCAMIDO PROPYL BETAINE | 1 à 10% |
| GLYCEROL | 1 à 10% |
| COCOGLUCOSIDEXI | 1 à 10% |
| SODIUM MYRETH SULFATE | 1 à 10% |
| CONSERVATEUR | 0 à 2% |
| DISTEARATEPEG6000 | 0 à 2% |
| POLYQUARTERNIUMJR400XI | 0 à 2% |
| PANTHENOL DEXTROGYRE | 0 à 2% |
| ACIDE CITRIQUE HYDXI | 0 à 2% |
| PERSEOSE AVOCAT | 0 à 1% |

### Crème solaire SPF 50+

**Tableau 7**

| Matière première / Nom commercial | de |
|---|---|
| CAPRYLATE/CAPRATEDECOPRAH | 5 à 20% |
| DICAPRYL YLCARBONATE | 5 à 20% |
| CAPRYLOCAPRATEGLYC | 5 à 20% |
| MIGLYOLGELB | 5 à 20% |
| TITANIDIOXIDJOJOBAESTERS | 1 à 15 % |
| TINOSORBS | 1 à 5% |
| DIETAMIN HYDBENZO YHEXBENZ | 1 à 10 % |
| ETHYL HEXYL TRIAZONE | 1 à 10 % |
| HUILE D'AVOCAT HAREF | 1 à 5% |
| ALPHA TOCOPHEROL | 0.05 à 1% |
| LAURYL GLUCOSE-GLYSTEARATXI | 2 à 12 % |
| EAU PURIFIEE | QSP 100% |
| GLYCEROL | 1 à 10 % |
| GOMME XANTHANE | 0 à 1% |
| CONSERVATEUR | 0 à 2% |
| POTASSIUM CETYL PHOSPHATE | 0 à 2% |
| LESSIVE SOUDEXI | 0 à 2% |
| PHENYL BENZIMIDAZO SULFONAC | 1 à 5% |
| PERSEOSE AVOCAT | 0 à 1% |

Les sucres d'avocat préparés par le procédé de l'exemple 1 et appelés « perséose d'avocat » ci-dessous ont été utilisés pour étudier l'expression des marqueurs de la fonction barrière dans différentes conditions.

### 2. Maturation épidermique

### a. Expression de l'involucrine

### Matériel et méthodes :

Des kératinocytes épidermiques humains normaux ont été incubés en présence de perséose d'avocat à 0,005% et 0,05% (p/v) pendant 48 heures.

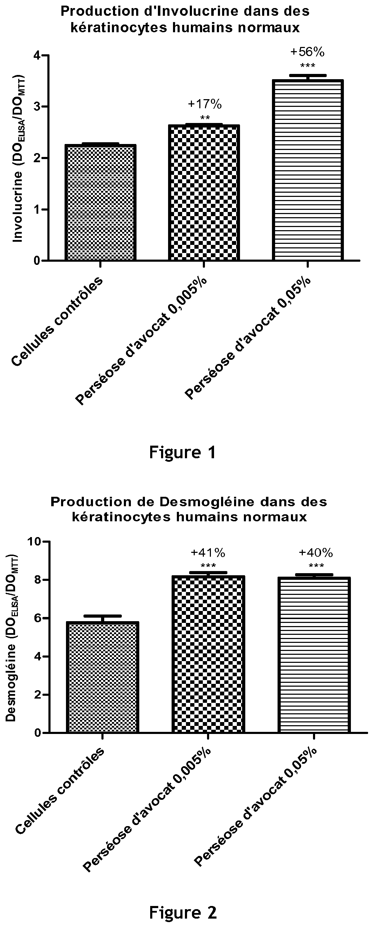

A la fin du traitement, l'involucrine produite a été dosée en surface des kératinocytes par une technique d'ELISA sur cellules. La quantité d'involucrine a été rapportée au nombre de cellules vivantes estimé par un dosage au MTT. Résultats : les résultats sont illustrés dans le tableau 8 ci-dessous, et dans la figure 1.

Le perséose d'avocat à 0,005% et 0,05% a significativement stimulé l'expression d'involucrine dans des kératinocytes humains normaux : respectivement +17%, p<0,01 et + 56%, p<0,001.

**Tableau 8**

| | Involucrine (DO_{ELISA}/DO_{MTT}) | |
|---|---|---|
| Cellules contrôles | 2,245 ± 0,068 | |
| Perséose d'avocat 0,005% | 2,633 ± 0,051 | + 17% ** |
| Perséose d'avocat 0,05% | 3,509 ± 0,178 | + 56% *** |

| | | |
|---|---|---|
| **p<0,01 ; *** p<0,001 vs cellules contrôles, Analyse de variance à un facteur suivie d'un test de Dunnett (logiciel GraphPad Prism) | | |

### b. Expression de la desmogléine

### Matériel et méthodes :

Des kératinocytes épidermiques humains normaux ont été cultivés dans un milieu induisant leur différenciation (supplémenté en Ca++) et incubés en présence de perséose d'avocat à 0,005% et 0,05% pendant 48 heures.

A la fin du traitement, la desmogléine-1 produite a été dosée en surface des kératinocytes par une technique d'ELISA sur cellules. La quantité de desmogléine a été rapportée au nombre de cellules vivantes estimé par un dosage au MTT.

Résultats : les résultats sont illustrés dans le tableau 9 ci-dessous, et dans la figure 2.

Le perséose d'avocat à 0,005% et 0,05% a significativement stimulé l'expression de la desmogléine 1 dans des kératinocytes humains normaux différenciés : respectivement +41%, p<0,001 et +40%, p<0,001.

**Tableau 9**

| | Desmogléine (DO_{ELISA}/DO_{MTT}) | |
|---|---|---|
| Cellules contrôles | 5,791 ± 0,641 | |
| Perséose d'avocat 0,005% | 8,182 ± 0,430 | + 41% *** |
| Perséose d'avocat 0,05% | 8,094 ± 0,395 | + 40% *** |

| | | |
|---|---|---|
| *** p<0,001 vs cellules contrôles, Analyse de variance à un facteur suivie d'un test de Dunnett (logiciel GraphPad Prism) | | |

### 3. Renforcement de la barrière lipidique : néosynthèse de céramides

### Matériel et méthodes :

Des épidermes reconstruits à J5 ont été cultivés dans les conditions suivantes :
- Epidermes contrôles : milieu déplété,
- Epidermes différenciés : milieu de différenciation complet,
- Perséose d'avocat à 0,005% et 0,05% (MS) dans le milieu déplété.

Après 24 heures d'incubation, le milieu de culture a été renouvelé et supplémenté avec de l'acétate radiomarqué (14C-acétate). Les épidermes ont ensuite été à nouveau incubés pendant 48 heures.

A la fin du traitement, les céramides néosynthétisés ont été analysés par quantification de la radioactivité incorporée après chromatographie sur couche mince.

Résultats : les résultats sont illustrés dans le tableau 10 ci-dessous.

Le perséose d'avocat à 0,005% et 0,05% a augmenté la synthèse de céramides dans des épidermes humains reconstruits : respectivement +32% et +27%.

**Tableau 10**

| | Quantité de céramides | |
|---|---|---|
| | Intensité relative (moyenne) | % du contrôle |
| Epidermes contrôles | 14,2 | 100 |
| Epidermes différenciés | 19,7 | 138 |
| Perséose d'avocat 0,005% | 18,0 | 132 |
| Perséose d'avocat 0,05% | 18,7 | 127 |

### 4. Régulation hydrique

### a. Activité enzymatique de la transglutaminase

### Matériel et méthodes :

Des kératinocytes épidermiques humains normaux ont été incubés en présence de perséose d'avocat à 0,005% et 0,05% dans du milieu KGM Gold (Lonza) ; un témoin de cellules cultivées en milieu pro-différenciant (KGM Gold supplémenté en Ca++) a été réalisé en parallèle.

Après 72h d'incubation, les cellules ont été lysées et l'activité de la transglutaminase intracellulaire dosée à l'aide du kit Transglutaminase Colorimetric Microassay (Covalab). Les résultats ont été normalisés par rapport à la quantité de protéines totales déterminée à l'aide du kit BC Assay (Interchim).

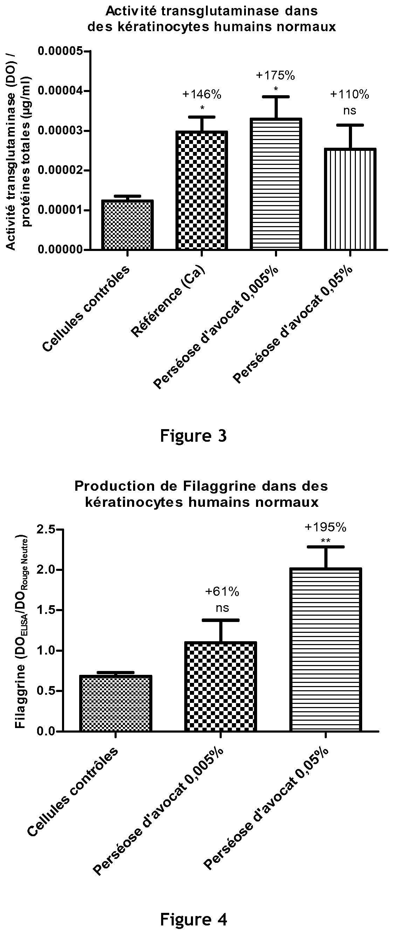

Résultats : les résultats sont illustrés dans le tableau 11 ci-dessous, et dans la figure 3.

Le perséose d'avocat à 0,005% a significativement stimulé l'activité enzymatique de la transglutaminase dans des kératinocytes épidermiques humains normaux : +175%, p<0,05.

**Tableau 11**

| | Activité transglutaminase (DO/protéines totales) | |
|---|---|---|
| Cellules contrôles | 0,000012 ± 0,0000018 | |
| Référence (Ca) | 0,00003 ± 0,0000054 | + 146% * |
| Perséose d'avocat 0,005% | 0,000033 ± 0,0000075 | + 175% * |
| Perséose d'avocat 0,05% | 0,000025 ± 0,0000086 | + 110% ns |

| | | |
|---|---|---|
| * p<0,05 vs cellules contrôles, Analyse de variance à un facteur suivie d'un test de Dunnett (logiciel GraphPad Prism) | | |

### b. Expression de la filaggrine

### Matériel et méthodes :

Des kératinocytes épidermiques humains normaux ont été incubés en présence de perséose d'avocat à 0,005% et 0,05% pendant 72 heures.

A la fin du traitement, la filaggrine produite a été dosée en surface des kératinocytes par une technique d'ELISA sur cellules. La quantité de filaggrine a été rapportée au nombre de cellules vivantes estimé par un dosage au Rouge Neutre. Résultats : les résultats sont illustrés dans le tableau 12 ci-dessous, et dans la figure 4.

Le perséose d'avocat à 0,05% a significativement stimulé la production de filaggrine par des kératinocytes épidermiques humains normaux : +195%, p<0,01.

**Tableau 12**

| | Filaggrine (DO_{ELISA}/DO_{RN}) | |
|---|---|---|
| Cellules contrôles | 0,683 ± 0,089 | |
| Perséose d'avocat 0,005% | 1,102 ± 0,681 | + 61% ns |
| Perséose d'avocat 0,05% | 2,014 ± 0,546 | + 195% ** |

| | | |
|---|---|---|
| ** p<0,01 vs cellules contrôles, Analyse de variance à un facteur suivie d'un test de Dunnett (logiciel GraphPad Prism). | | |

### c. Expression génique de l'enzyme PAD1 (Peptidyl Arginin Deiminase)

### Matériel et méthodes :

Des kératinocytes épidermiques humains normaux, cultivés dans un milieu induisant la différenciation (supplémenté en Ca++), ont été incubés pendant 48 heures en présence de perséose d'avocat à 0,005% et 0,05% ou de vitamine D3 à 10-7M (1α,25-Dihydroxyvitamin D3 ; Sigma), référence positive.

A la fin du traitement, les ARN ont été extraits et l'expression génique de PAD1 a été évaluée par RT-PCR en temps réel, le gène de référence GAPDH a été utilisé pour normaliser les résultats.

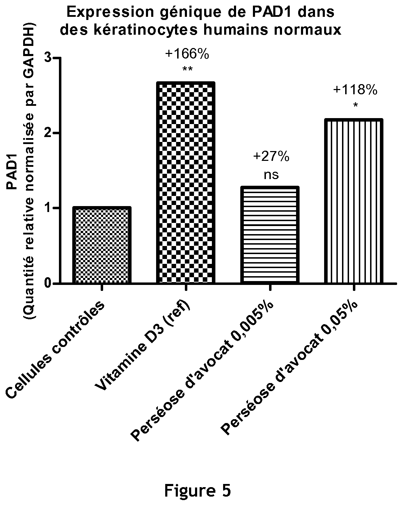

Résultats : les résultats sont illustrés dans le tableau 13 ci-dessous, et dans la figure 5.

Le perséose d'avocat à 0,05% a significativement stimulé l'expression génique de PAD1 : +118%, p<0,05.

**Tableau 13**

| | PAD1 (Quantité Relative normalisée par GAPDH) | |
|---|---|---|
| Cellules contrôles | 1,00 | |
| Vitamine D3 10⁻⁷M | 2,66 | +166% ** |
| Perséose d'avocat 0,005% | 1,27 | + 27% ns |
| Perséose d'avocat 0,05% | 2,18 | + 118% * |

| | | |
|---|---|---|
| *p<0,05 ; ** p<0,01 vs cellules contrôles, Analyse de variance à un facteur suivie d'un test de Dunnett (logiciel GraphPad Prism) | | |

### d. Production d'acide hyaluronique

### Matériel et méthodes :

Des kératinocytes épidermiques humains normaux ont été incubés en présence de perséose d'avocat à 0,005% et 0,05% ou d'acide rétinoïque à 10-7M (all-trans-Retinoic acid ; Sigma), référence positive.

Après 24 heures d'incubation, l'acide hyaluronique produit par les kératinocytes a été dosé dans les surnageants de culture par une méthode ELISA (Tebu). La quantité d'acide hyaluronique mesurée a été rapportée au nombre de cellules vivantes estimé par un test au Rouge Neutre.

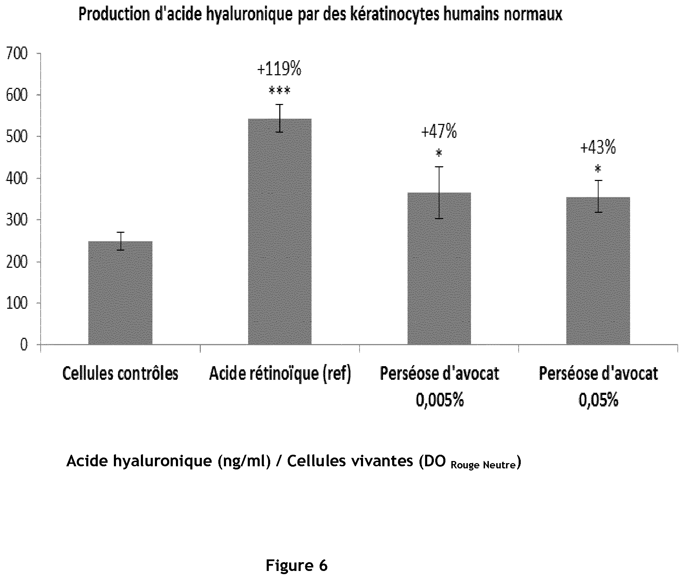

Résultats : les résultats sont illustrés dans le tableau 14 ci-dessous, et dans la figure 6.

Le perséose d'avocat a significativement augmenté la quantité d'acide hyaluronique produit par des kératinocytes humains normaux : +47%, p<0,05 à 0,005% et +43%, p<0,05 à 0,05%.

**Tableau 14**

| | Acide hyaluronique (ng/ml / Cellules vivantes) | |
|---|---|---|
| Cellules contrôles | 248,445 ± 21,248 | |
| Ac. rétinoique 10⁻⁷M | 544,024 ± 34,026 | +119% *** |
| Perséose d'avocat 0,005% | 365,737 ± 61,68 | + 47% * |
| Perséose d'avocat 0,05% | 356,179 ± 37,353 | + 43% * |

| | | |
|---|---|---|
| *p<0,05 ; *** p<0,001 vs cellules contrôles, Analyse de variance à un facteur suivie d'un test de Dunnett (logiciel GraphPad Prism). | | |

### 5. Modèle d'agression UV

Certaines agressions environnementales sont néfastes pour la maturation de l'épiderme et notamment pour la fonction barrière (Yamamoto T, Kurasawa M, Hattori T, Maeda T, Nakano H, Sasaki H. Relationship between expression of tight junction-related molecules and perturbed epidermal barrier function in UVB-irradiated hairless mice. Arch Dermatol Res 2008; 300: 61-8 ; Valacchi et al. Cutaneous responses to environmental stressors. Ann NY Acad Sci 2012; 1271: 75-81, 2012).

### Matériel et méthodes :

Des kératinocytes épidermiques humains normaux, cultivés en milieu induisant la différenciation (supplémenté en Ca++), ont été pré-incubés en présence de perséose d'avocat à 0,001% pendant 48 heures ou à 0,005% et 0,05% pendant 24 heures. Les kératinocytes ont ensuite été irradiés, dans du PBS, par les UVA+B à la dose de 36 kJ/m². Les cellules ont alors été à nouveau incubées pendant 6 heures en présence de perséose d'avocat.

### A la fin du traitement, les ARN ont été extraits et les expressions géniques de la claudine 1, de la filaggrine et de l'involucrine ont été évaluées par RT-PCR en temps réel. Les résultats ont été normalisés par amplification d'un gène de référence : β2-microglobuline pour les essais à 0,001% de perséose d'avocat ou HPRT pour les essais à 0,005% et 0,05% de perséose d'avocat.

Résultats : les résultats sont illustrés dans le tableau 15 ci-dessous.

L'irradiation aux UV a induit une diminution significative de l'expression génique des trois marqueurs de barrière étudiés.

Le perséose d'avocat a permis de contrebalancer l'effet des UV en stimulant significativement l'expression génique de la claudine 1 (+131%, p<0,001 à 0,001% et 32%, p<0,01 à 0,05%), de la filaggrine (+22%, p<0,05 à 0,005% et +27%, p<0,01 à 0,05%) ainsi que de l'involucrine (+191%, p<0,01 à 0,001%) sous UV.

**Tableau 15**

| | Claudine 1 | | | | Filaggrine | | Involucrine | |
|---|---|---|---|---|---|---|---|---|
| Cellules contrôles | 1,00 | | 1,00 | | 1,00 | | 1,00 | |
| Témoin irradié UVA+B 36kJ/m² | 0,39 | -61% $$$ | 0,50 | -50% $$$ | 0,56 | -44% $$$ | 0,29 | -71% $$$ |
| Perséose d'avocat 0,001% | 0,90 | +131%*** | / | | / | | 0,85 | +191%** |
| Perséose d'avocat 0,005% | / | | 0,60 | +20% ns | 0,69 | +22% * | / | |
| Perséose d'avocat 0,05% | / | | 0,66 | +32% ** | 0,71 | +27% ** | / | |

| | | | | | | | | |
|---|---|---|---|---|---|---|---|---|
| $$$ p<0,001 vs cellules contrôles * p<0,05 ; ** p<0,01 ; *** p<0,001 vs témoin irradié | | | | | | | | |

Analyse de variance à un facteur suivie d'un test de Tukey (logiciel GraphPad Prism).

## Revendications

1. Méthode pour évaluer l'efficacité d'un actif choisi parmi les sucres en C7 et les dérivés de formule (I), 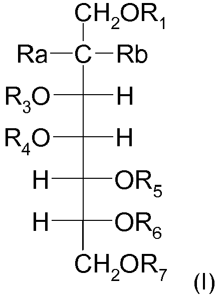 où
Ra représente un atome d'hydrogène et Rb représente un -OR2 ou CRaRb représente le radical CO ;
R1, R2, R3, R4, R5, R6 et R7 représentent, indépendamment l'un de l'autre
- un atome d'hydrogène ou
- un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique ; ou
- un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy (-OC2H5) et groupement -SO3M avec M représentant un atome d'hydrogène, un ion ammonium NH4+ ou un ion métallique ;
dans la prévention et/ou le traitement d'au moins une déficience de la barrière cutanée d'un sujet, ladite méthode comprenant les étapes de :
a. mesure du niveau d'expression et/ou d'activité enzymatique d'une combinaison de marqueurs biologiques dans un échantillon de cellules cutanées du sujet, **caractérisé en ce que** ladite combinaison de marqueurs comprend :
- au moins un marqueur de la maturation épidermique choisi parmi la desmogléine 1 et l'involucrine ;
- au moins un marqueur de la barrière lipidique choisi parmi les céramides, en particulier parmi les céramides 1 à 9, encore plus particulièrement le céramide 1 ;
- au moins un marqueur de la régulation hydrique choisi parmi la filaggrine, Peptidyl Arginine Deiminase Type 1 (PAD1), l'acide hyaluronique et la transglutaminase 1 ; et
- au moins un marqueur de la régulation du *stratum granulosum* choisi parmi les claudines, en particulier la claudine 1 ;
b. mesure du niveau d'expression et/ou d'activité enzymatique de ladite combinaison de marqueurs biologiques dans un échantillon de cellules cutanées de référence;
c. comparaison des niveaux d'expression et/ou d'activité enzymatique obtenus à l'étape a) avec des niveaux d'expression et/ou d'activité enzymatique obtenus à l'étape b);
d. d'évaluation de l'efficacité dudit actif en fonction de la comparaison de l'étape b) ;
dans laquelle ledit sujet est un enfant, préférentiellement un nouveau-né, c'est-à-dire un enfant dont l'âge est compris entre 0 et 1 mois, un nourrisson, c'est-à-dire un enfant dont l'âge est compris entre 1 mois et 2 ans, ou un enfant de 2 ans ou plus, c'est-à-dire un enfant dont l'âge est compris entre 2 ans et 16 ans ; et
dans laquelle ledit niveau d'activité enzymatique s'applique aux marqueurs biologiques pour lesquels une activité enzymatique peut être mesurée.

2. Méthode selon la revendication 1, **caractérisée en ce que** la combinaison de marqueurs biologiques consiste en la desmogléine 1, l'involucrine, le céramide 1, la filaggrine, PAD1, l'acide hyaluronique, la transglutaminase 1 et la claudine 1.

3. Méthode selon la revendication 1 ou 2, **caractérisée en ce que** ledit échantillon de cellules cutanées de référence est un échantillon de cellules cutanées dudit sujet n'ayant pas été traitées par ledit actif.

4. Méthode selon la revendication 1 ou 2, **caractérisée en ce que** ledit échantillon de cellules cutanées de référence est un échantillon de cellules cutanées d'un sujet présentant ladite déficience de la barrière cutanée, ledit sujet n'ayant pas été traité pour ladite déficience.

5. Méthode selon la revendication 1 ou 2, **caractérisée en ce que** ledit échantillon de cellules cutanées référence est un échantillon de cellules cutanées d'un sujet sain.

6. Méthode selon l'une des revendications 1 à 5, **caractérisée en ce que** l'échantillon de cellules cutanées de référence est un échantillon de cellules cutanées d'un enfant, préférentiellement d'un nouveau-né, c'est-à-dire d'un enfant dont l'âge est compris entre 0 et 1 mois, d'un nourrisson, c'est-à-dire d'un enfant dont l'âge est compris entre 1 mois et 2 ans, ou d'un enfant de 2 ans ou plus, c'est-à-dire d'un enfant dont l'âge est compris entre 2 ans et 16 ans.

7. Méthode selon l'une des revendications 1 à 6, **caractérisée en ce que** ladite déficience de la barrière cutanée est choisie parmi l'acné du nourrisson, l'acné de l'adolescent, la rosacée ou érythrocouperose, le psoriasis, la dermite du siège, la dermatite atopique, l'eczéma, la dermatite de contact, la dermatite irritative et en particulier la dermatite irritative du siège ou érythème fessier, la dermatite allergique, la dermite séborrhéique, la peau sensible, la peau réactive, la xérose, la peau déshydratée, la peau endommagée par le soleil, par les radiations, par le vent, par le froid, par le chaud, par le stress, par la pollution, l'érythème cutané, la peau âgée ou photoâgée, la peau photosensibilisée, les dartres, les ichtyoses, les gerçures, les brûlures, les coups de soleil, les inflammations dus aux rayons de toutes sortes, les irritations par agents chimiques, physiques, bactériologiques, fongiques ou viraux, parasitaires.

8. Méthode selon l'une des revendications 1 à 7, **caractérisée en ce que** le niveau d'expression du marqueur choisi parmi la desmogléine 1, l'involucrine, ou la filaggrine, correspond au niveau d'expression dudit marqueur sous sa forme peptidique, le niveau d'expression de PAD1 ou de la claudine 1 correspond au niveau d'expression dudit marqueur sous sa forme nucléotidique.

9. Méthode selon l'une des revendications 1 à 8, **caractérisée en ce que** ledit actif est le D-mannoheptulose, de formule générale (II)

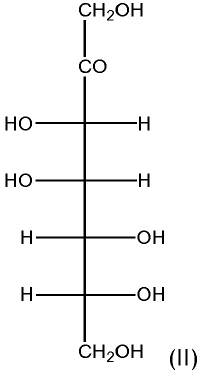

10. Méthode selon l'une des revendications 1 à 8, **caractérisée en ce que** ledit actif est le perséitol, de formule générale (III)

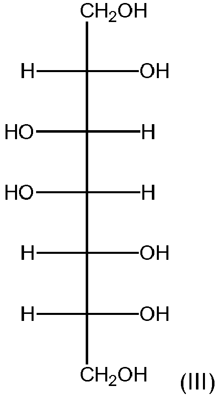

11. Méthode selon l'une des revendications 1 à 10, **caractérisée en ce que** ledit actif est sous la forme d'une composition, préférentiellement adaptée à une administration topique.

12. Méthode selon la revendication 11, **caractérisée en ce que** la composition peut en outre comprendre au moins un autre composé actif en plus des sucres en C7 et des dérivés de formule (I).

## Patentansprüche

1. Verfahren zum Bewerten der Wirksamkeit eines Wirkstoffs, der unter C7-Zucker und Derivaten der Formel (I) ausgewählt ist, 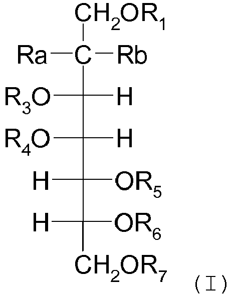 wobei
Ra ein Wasserstoffatom darstellt und Rb ein -OR2 darstellt oder CRaRb den Rest CO darstellt;
R1, R2, R3, R3, R3, R4, R5, R6 und R7 unabhängig voneinander Folgendes darstellen
- ein Wasserstoffatom oder
- einen Rest -(CO)-R, wobei R eine gesättigte oder ungesättigte Kohlenwasserstoffkette mit 11 bis 24 Kohlenstoffatomen darstellt, die gegebenenfalls durch einen oder mehrere Substituenten substituiert ist, die aus der Gruppe bestehend aus Hydroxy-(-OH)-Resten, Ethoxy(-OC2H5)-Resten und -SO3M-Gruppen ausgewählt sind, wobei M ein Wasserstoffatom, ein Ammonium NH4+-Ion oder ein Metallion darstellt; oder
- einen Rest -(CO)-R', wobei R' eine gesättigte oder ungesättigte Kohlenwasserstoffkette mit 2 bis 10 Kohlenstoffatomen darstellt, die gegebenenfalls durch einen oder mehrere Substituenten substituiert ist, die aus der Gruppe bestehend aus Hydroxy-(-OH)-Resten, Ethoxy(-OC2H5)-Resten und -SO3M-Gruppe ausgewählt ist, wobei M ein Wasserstoffatom, ein Ammonium NH4+-Ion oder ein Metallion darstellt;
bei der Vorbeugung und/oder Behandlung zumindest eines Mangels der Hautbarriere eines Probanden, wobei das Verfahren die folgenden Schritte umfasst:
a. Messen des Grads der Expression und/oder der Enzymaktivität einer Kombination von biologischen Markern in einer Probe von Hautzellen des Probanden, **dadurch gekennzeichnet, dass** die Markerkombination Folgendes umfasst:
- zumindest einen Marker für die epidermale Reifung, der unter Desmoglein 1 und Involucrin ausgewählt ist;
- zumindest einen Marker der Lipidbarriere, der unter Ceramiden, insbesondere aus den Ceramiden 1 bis 9, insbesondere Ceramid 1 ausgewählt ist;
- zumindest einen Marker für die Regulierung des Wasserhaushaltes, der unter Filaggrin, Peptidyl-Arginin-Deiminase Typ 1 (PAD1), Hyaluronsäure und Transglutaminase 1 ausgewählt ist; und
- zumindest einen Marker für die *Stratum granulosum* Regulierung, der unter Claudinen, insbesondere Claudin 1 ausgewählt ist;
b. Messen des Expressions- und/oder Enzymaktivitätsgrads der Kombination von biologischen Markern in einer Probe von Referenzhautzellen;
c. Vergleich der in Schritt a) erhaltenen Expressions- und/oder Enzymaktivitätsgrads mit den in Schritt b) erhaltenen Expressions- und/oder Enzymaktivitätsgrads;
d. Bewertung der Wirksamkeit des genannten Wirkstoffs auf der Grundlage des Vergleichs in Schritt b) ;
wobei die Person ein Kind ist, vorzugsweise ein Neugeborenes, d. h. ein Kind, dessen Alter zwischen 0 und 1 Monat liegt, ein Säugling, d. h. ein Kind, dessen Alter zwischen 1 Monat und 2 Jahren liegt, oder ein Kind mit 2 Jahren oder darüber, d. h. ein Kind, dessen Alter zwischen 2 und 16 Jahren liegt; und
wobei der Enzymaktivitätsgrad für biologische Marker gilt, bei denen eine Enzymaktivität gemessen werden kann.

2. Verfahren nach Anspruch 1, **dadurch gekennzeichnet, dass** die Kombination von biologischen Markern aus Desmoglein 1, Involucrin, Ceramid 1, Filaggrin, PAD1, Hyaluronsäure, Transglutaminase 1 und Claudin 1 besteht.

3. Verfahren nach Anspruch 1 oder 2, **dadurch gekennzeichnet, dass** die Referenz-Hautzellenprobe eine Probe von Hautzellen des Patienten ist, die nicht mit dem Wirkstoff behandelt wurden.

4. Verfahren nach Anspruch 1 oder 2, **dadurch gekennzeichnet, dass** die Referenz-Hautzellenprobe eine solche eines Probanden mit Hautbarrieremangel ist, wobei der Proband nicht wegen des Mangels behandelt wurde.

5. Verfahren nach Anspruch 1 oder 2, **dadurch gekennzeichnet, dass** die Referenz-Hautzellenprobe eine Hautzellenprobe eines gesunden Probanden ist.

6. Verfahren nach einem der Ansprüche 1 bis 5, **dadurch gekennzeichnet, dass** die Referenz-Hautzellenprobe eine Hautzellenprobe eines Kindes, vorzugsweise eines Neugeborenen, d. h. eines Kindes, dessen Alter zwischen 0 und 1 Monat liegt, eines Säuglings, d. h. eines Kindes, dessen Alter zwischen 1 Monat und 2 Jahren liegt, oder eines Kindes von 2 Jahren oder mehr ist, d. h. eines Kindes, dessen Alter zwischen 2 Jahren und 16 Jahren liegt.

7. Verfahren nach einem der Ansprüche 1 bis 6, **dadurch gekennzeichnet, dass** der Hautbarrieremangel unter Säuglingsakne, jugendlicher Akne, Rosacea oder Erythrocouperose, Psoriasis, Windeldermatitis, atopischer Dermatitis, Ekzemen, Kontaktdermatitis, irritativer Dermatitis und insbesondere irritativer Windeldermatitis oder Windelausschlag, allergischer Dermatitis, seborrhoischer Dermatitis, empfindlicher Haut, reaktiver Haut, Xerose, dehydrierter Haut, durch Sonne, Strahlung, Wind, Kälte, Hitze, Stress, Umweltverschmutzung, Hautrötung beschädigter Haut, alter oder lichtgealterter Haut, lichtsensibilisierter Haut, Flechten, Ichthyosen, rissiger Haut, Verbrennungen, Sonnenbrand, Entzündungen durch Strahlen aller Art, Reizungen durch chemische, physikalische, bakteriologische, Pilz- oder virale Mittel, Parasiten ausgewählt ist.

8. Verfahren nach einem der Ansprüche 1 bis 7, **dadurch gekennzeichnet, dass** der Expressionsgrad des unter Desmoglein 1, Involucrin oder Filaggrin ausgewählten Markers dem Expressionsgrad des Markers in seiner Peptidform entspricht, der Expressionsgrad von PAD1 oder Claudin 1 dem Expressionsgrad des Markers in seiner Nucleotidform entspricht.

9. Verfahren nach einem der Ansprüche 1 bis 8, **dadurch gekennzeichnet, dass** der Wirkstoff D-Mannoheptulose der allgemeinen Formel (II) ist.

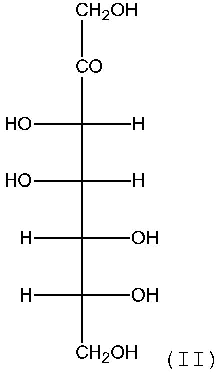

10. Verfahren nach einem der Ansprüche 1 bis 8, **dadurch gekennzeichnet, dass** der Wirkstoff Perseitol der allgemeinen Formel (III) ist.

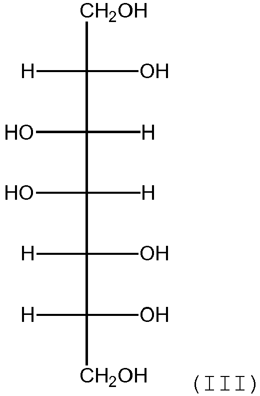

11. Verfahren nach einem der Ansprüche1 bis 10, **dadurch gekennzeichnet, dass** der Wirkstoff in Form einer Zusammensetzung vorliegt, die vorzugsweise für die topische Verabreichung geeignet ist.

12. Verfahren nach Anspruch 11, **dadurch gekennzeichnet, dass** die Zusammensetzung ferner zumindest einen weiteren Wirkstoff zusätzlich zu C7-Zucker und Derivaten der Formel (I) umfassen kann.

## Claims

1. A method for assessing the effectiveness of an active ingredient selected from C7 sugars and derivatives of formula (I), 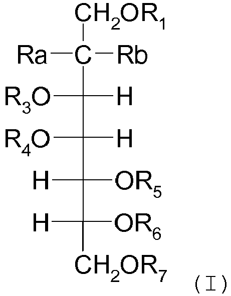 where
Ra is hydrogen and Rb is -OR2 or CRaRb is the CO radical;
R1, R2, R3, R4, R5, R6 and R7 are, independently from each other:
- hydrogen or
- a -(CO)-R radical wherein R is a saturated or unsaturated hydrocarbon chain containing from 11 to 24 carbon atoms, optionally substituted by one or more substituent(s) selected from the group consisting of hydroxyl radicals (-OH), ethoxy radicals (-OC2H5) and the -SO3M group with M being hydrogen, an ammonium ion (NH4+) or a metal ion; or
- a -(CO)-R' radical wherein R' is a saturated or unsaturated hydrocarbon chain containing from 2 to 10 carbon atoms, optionally substituted by one or more substituent(s) selected from the group consisting of hydroxyl radicals (-OH), ethoxy radicals (-OC2H5) and the -SO3M group with M being hydrogen, an ammonium ion (NH4+) or a metal ion;
in the prevention and/or treatment of at least one deficiency of the skin barrier of a subject, said method comprising the steps of:
a. measuring the level of expression and/or enzymatic activation of a combination of biological markers in a sample of skin cells from the subject, **characterized in that** said combination of markers comprises:
- at least one marker of epidermal maturation selected from desmoglein 1 and involucrin;
- at least one marker of the lipid barrier selected from ceramides, in particular ceramides 1 to 9, more particularly ceramide 1;
- at least one marker of water regulation selected from filaggrin, Peptidyl Arginine Deiminase type 1 (PAD1), hyaluronic acid, and transglutaminase 1; and
- at least one marker of regulation of the *stratum granulosum* selected from claudins, in particular claudin 1;
b. measuring the level of expression and/or enzymatic activation of said biological marker combination in a reference skin cell sample;
c. comparing the levels of expression and/or enzymatic activation obtained in step a) with levels of expression and/or enzymatic activation obtained in step b) ;
d. evaluating the effectiveness of said active ingredient based on the comparison of step b).
wherein said subject is a child, preferably a newborn, that is to say a child between 0 and 1 month of age, a baby, that is to say a child between 1 month and 2 years of age, or a child of 2 years or more, that is to say a child between 2 and 16 years of age; and
wherein said level of enzymatic activation applies to biological markers for which an enzymatic activation can be measured.

2. The method according to claim 1, **characterized in that** the combination of biological markers consists of desmoglein 1, involucrin, ceramide 1, filaggrin, PAD1, hyaluronic acid, transglutaminase 1 and claudin 1.

3. The method according to claim 1 or 2, **characterized in that** said reference skin cell sample is a sample of skin cells from said subject not having been treated with said active ingredient.

4. The method according to claim 1 or 2, **characterized in** said reference skin cell sample is a sample of skin cells from a subject presenting said deficiency of the skin barrier, said subject not having been treated for said deficiency.

5. The method according to claim 1 or 2, **characterized in that** said reference skin cell sample is a sample of skin cells from a healthy subject.

6. The method according to any one of claims 1 to 5, **characterized in that** the reference skin cell sample is a sample of skin cells from a child, preferably a newborn, that is to say a child between 0 and 1 month of age, a baby, that is to say a child between 1 month and 2 years of age, or a child of 2 years or more, that is to say a child between 2 and 16 years of age.

7. The method according to any one of claims 1 to 6, **characterized in that** said deficiency of the skin barrier is selected from baby acne, adolescent acne, rosacea or erythrocouperosis, psoriasis, diaper dermatitis, atopic dermatitis, eczema, contact dermatitis, irritant dermatitis and in particular irritant diaper dermatitis or diaper rash, allergic dermatitis, seborrheic dermatitis, sensitive skin, reactive skin, xerosis, dry skin, skin damaged by the sun, by radiation, by wind, by cold, by heat, by stress, by pollution, cutaneous erythema, aged or photoaged skin, photosensitive skin, scurf, ichthyoses, chapping, burns, sunburns, inflammations due to rays of all kinds, irritations by chemical, physical, bacteriological, fungal or viral, or parasitic agents.

8. The method according to any one of claims 1 to 7, **characterized in that** the level of expression of the marker selected from desmoglein 1, involucrin or filaggrin corresponds to the level of expression of said marker in its peptide form; the level of expression of PAD1 or of claudin 1 corresponds to the level of expression of said marker in its nucleotide form.

9. The method according to any one of claims 1 to 8, **characterized in that** said active ingredient is D-mannoheptulose, of general formula (II)

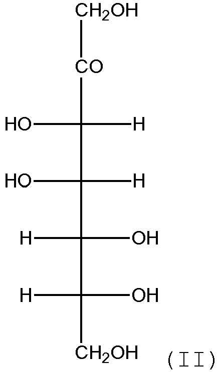

10. The method according to any one of claims 1 to 8, **characterized in that** said active ingredient is perseitol, of general formula (III)

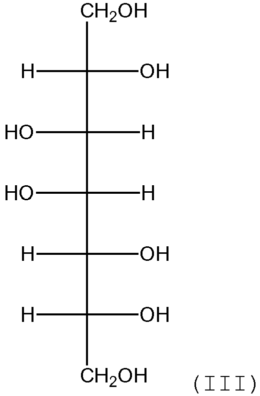

11. The method according to any one of claims 1 to 10, **characterized in that** said active ingredient is in the form of a composition, preferably suitable for topical application.

12. The method according to claim 11, **characterized in that** the composition further comprises at least one other active compound in addition to C7 sugars and derivatives of formula (I).
